# EUROPEAN PATENT SPECIFICATION

(11) **EP 3 494 989 B1**
(45) Date of publication and mention of the grant of the patent: **16.07.2025**
(21) Application number: 18204631.8
(22) Date of filing: 25.01.2013
(51) Int. Cl.: A61K 39/145

(54) **COMPOSITE ANTIGENIC SEQUENCES AND VACCINES**
ZUSAMMENGESETZTE ANTIGENSEQUENZEN UND IMPFSTOFFE
SÉQUENCES ET VACCINS ANTIGÉNIQUES COMPOSITES

(30) Priority: 26.01.2012 US 201261591113 P
(43) Date of publication of application: 12.06.2019
(62) Divisional of application: 13741334.0
(73) Proprietor: Longhorn Vaccines and Diagnostics, LLC, Bethesda, MD 20814 (US)
(72) Inventor: FISCHER, Gerald W., Bethesda, MD 20817 (US); DAUM, Luke T., San Antonio, TX 78209 (US)
(74) Representative: Forresters IP LLP

(56) References cited:
- WO-A1-2009/029686
- WO-A1-2010/056796
- WO-A2-2009/016639
- WO-A2-97/44469

## Description

### Background

### 1 Field of the Invention

The present invention is directed to composite antigens comprising SEQ ID NO:66 or 67, and to tools and the composite antigen for use in methods for generating an immune response with the composite antigens of the invention.

### 2 Description of the Background

Microbial and viral pathogens are a primary source of infectious disease in animals. Pathogens and their hosts constantly adapt to one another in an endless competition for survival and propagation. Certain pathogens have become enormously successful at infecting mammalian hosts and surviving exposure to the host immune response, even over periods of years or decades. One example of an extremely successful mammalian pathogen is the influenza virus.

Influenza viruses are etiologic agents for a contagious respiratory illness (commonly referred to as the flu) that primarily affects humans and other vertebrates. Influenza is highly infectious and an acute respiratory disease that has plagued the human race since ancient times. Infection is characterized by recurrent annual epidemics and periodic major worldwide pandemics. Influenza virus infection can cause mild to severe illness, and can even lead to death. Every year in the United States, 5 to 20 percent of the population, on average, contracts the flu with more than 200,000 hospitalizations from complications and over 36,000 deaths. Because of the high disease-related morbidity and mortality, direct and indirect social economic impacts of influenza are enormous. Four pandemics occurred in the last century, together causing tens of millions of deaths worldwide.

Influenza virus spreads from host to host through coughing or sneezing. Airborne droplets are the primary transmission vectors between individuals. In humans, the virus typically spreads directly from person to person, although persons can also be infected from indirect contact with surfaces harboring the virus. Infected adults become infectious to others beginning as little as one day before primary symptoms of the disease develop. Thereafter, these persons remain infectious for up to 5 days or more after. Uncomplicated influenza illness is often characterized by an abrupt onset of constitutional and respiratory symptoms, including fever, myalgia, headache, malaise, nonproductive cough, sore throat, rhinitis, or a combination of one or more of these symptoms.

Currently, the spread of pathogenic influenza virus is controlled in animal populations by vaccination and/or treatment with one or more anti-viral compounds. Vaccines containing inactivated influenza virus or simply influenza antigen are currently in use worldwide and especially promoted for use by high-risk groups such as infants, the elderly, those without adequate health care and immunocompromised individuals. Most all viruses for vaccine use are propagated in fertile hen's eggs, inactivated by chemical means, and the antigens purified. The vaccines are usually trivalent, containing representative influenza A viruses (H1N1 and H3N2) and influenza B strains. The World Health Organization (WHO) regularly updates the specific strains targeted for vaccine development to those believed to be most prevalent and thereby maximize overall world efficacy. During inter-pandemic periods, it typically takes eight months or more before an updated influenza vaccine is ready for market. Historically, viral pandemics are spread to most continents within four to six months, and future viral pandemics are likely to spread even faster due to increased international travel. It is likely inevitable that an effective vaccine made by conventional means will be unavailable or in very short supply during the first wave of any future widespread outbreak or pandemic.

Pandemic flu can and does arise at any time. Although the severity of the next Influenza pandemic cannot be accurately predicted, modeling studies suggest that the impact of a pandemic on the United States, and the world as a whole, would be substantial. In the absence of any control measures (vaccination or drugs), it has been estimated that in the United States a "medium-level" pandemic could cause: 89,000 to 207,000 deaths; 314,000 and 734,000 hospitalizations; 18 to 42 million outpatient visits; and another 20 to 47 million people being sick. According to the Centers for Disease Control and Prevention (CDC) (Atlanta, GA, USA), between 15 percent and 35 percent of the U.S. population could be affected by an influenza pandemic, and the economic impact could range between approximately $71 and $167 billion.

Vaccines capable of producing a protective immune response have been produced in the last half century. These include whole virus vaccines, split-virus vaccines, and surface-antigen vaccines and live attenuated virus vaccines. While formulations of any of these vaccine types are capable of producing a systemic immune response, live attenuated virus vaccines have the advantage of also being able to stimulate local mucosal immunity in the respiratory tract.

With the continual emergence (or re-emergence) of different influenza strains, new influenza vaccines are continually being developed. Because of the rapid mutation rate among Influenza viruses, it has been extremely difficult and at times not possible to identify the antigenic moieties of the emergent virus strains in sufficient time to develop a suitable vaccine. Thus, polypeptides and polynucleotides of newly emergent or re-emergent virus strains (especially sequences of antigenic genes) are highly desirable.

Influenza is typically caused by infection of two genera of influenza viruses: Influenzavirus A and Influenzavirus B. The third genus of influenza viruses, Influenzavirus C, exists as a single species, influenza C virus, which causes only minor common cold-like symptoms in susceptible mammals. Infections by influenza A virus and influenza B virus are typically initiated at the mucosal surface of the upper respiratory tract of susceptible mammals. Viral replication is primarily limited to the upper respiratory tract but can extend to the lower respiratory tract and cause bronchopneumonia that can be fatal.

Influenza A virus, in particular, has many different serotypes. Presently, there are sixteen known variations of HA (the hemaglutination antigen which is involved in virus to cell binding) and nine known variations of NA (the neuraminidase antigen which is involved in viral release) within influenza A viruses, thus yielding 144 possible "HN" serotypes of influenza A virus based on variations within these two proteins alone. Only a small number of these combinations are believed to be circulating within susceptible populations at any given time. Once a new influenza strain or serotype emerges and spreads, the historical pattern is that it becomes established within the susceptible population and then moves around or "circulates" for many years causing seasonal epidemics of the Flu.

Three genera of influenza viruses currently comprise the Orthomyxoviridae Family: Influenza virus A, Influenza virus B, and Influenza virus C. Each of these genera contains a single species of influenza virus: The genus Influenza virus A consists of a single species, influenza A virus, which includes all of the influenza virus strains currently circulating among humans, including, for example, but not limited to, H1N1, H1N2, H2N2, H3N1, H3N2, H3N8, H5N1, H5N2, H5N3, H5N8, H5N9, H7N1, H7N2, H7N3, H7N4, H7N7, H9N2, and H10N7 serotypes. Exemplary influenza A viral strains include, but are not limited to, A/Aichi/2/68, A/Alaska/6/77, A/Alice, A/Ann Arbor/6/60, A/Bayern/7/95, A/Beijing/352/89, A/Beijing/353/89, A/Bethesda/1/85, A/California/10/78, A/Chick/Germany/N/49, A/Chile/1/83, A/Denver/1/57, A/Dunedin/6/83, A/Equine/Miami/1/63, A/FM/1/47, A/Great Lakes/0389/65, A/Guizhou/54/89, A/Hong Kong/77, A/Hong Kong/8/68, A/Hong Kong/483/97, A/Johannesburg/33/94, A/Kawasaki/9/86, A/Kiev/59/79, A/Korea/1/82, A/Korea/426/68, A/Leningrad/13/57, A/Los Angeles/2/87, A/MaI/302/54, A/Memphis/8/88, A/Nanchang/933/95, A/New Jersey/8/76, A/NT/60/68, A/NWS/33, A/Peking/2/79, A/Port Chalmers/1/73, A/PR/8/34, A/Shanghai/11/87, A/Shanghai/16/89, A/Shanghai/31/80, A/Singapore/1/57, A/Singapore/6/86, A/South Carolina/1/181918, A/Swine/1976/31, A/Swine/Iowa/15/30, A/Swine/New Jersey/8/76, A/Sydney/5/97, A/Taiwan/1/86, A/Taiwan/1/86A1, A/Texas/35/91, A/Texas/36/91, A/USSR/90/77, A/Victoria/3/75, A/Vietnam/1203/04, A/Washington D.C./897/80, A/Weiss/43, A/WS/33, A/WSN/33, A/Wuhan/359/95, A/Wyoming/1/87, and A/Yamagata/32/89, as well as derivatives, variants, and homologs thereof.

The genus Influenza virus B consists of a single species, influenza B virus, of which there is currently only one known serotype. Influenza B virus is almost exclusively a human pathogen, but is significantly less common and less genetically diverse than influenza A strains. Because of this limited genetic diversity, most humans acquire a certain degree of immunity to influenza B virus at an early age; however, the mutation frequency of the virus is sufficiently high enough to prevent lasting immunity by most humans, but not high enough to permit pandemic infection by influenza B virus across human populations. Exemplary influenza B viral serotypes include, but are not limited to, B/Allen/45, B/Ann Arbor/1/86, B/Bangkok/163/90, B/Beijing/184/93, B/Brigit, B/GL/1739/54, B/Hong Kong/330/2001, B/Hong Kong/5/72, B/Lee/40, B/Maryland/1/59, B/Mass/3/66, B/Oman/16296/2001, B/Panama/45/90, B/R22 Barbara, B/R5, B/R75, B/Russia/69, B/Shandong/7/97, B/Sichuan/379/99, B/Taiwan/2/62, B/Tecumseh/63/80, B/Texas/1/84, B/Victoria/2/87, and B/Yamagata/16/88, as well as derivatives, variants, and homologs thereof.

The genus Influenza virus C also consists of a single species, denoted influenza C virus, of which there is also currently only one known serotype. This serotype is known to infect both primates and porcines, and while infections of influenza C virus are rare, the resulting illness can be severe. Epidemics of influenza C virus are not uncommon in exposed populations, however, due to its rapid transmissibility in humans having close contact.

Polynucleotide and polypeptide sequences from each of these strains are contained within the publicly-available databases of the National Center for Biotechnology Information (National Library of Medicine, National Institutes of Health, Bethesda, MD, USA), and viral stocks may be obtained from the American Type Culture Collection (Manassas, VA, USA), or are otherwise publicly available.

Human influenza virus usually refers to influenza virus serotypes that are transmissible among humans. There are only three known influenza A virus HN serotypes that have circulated widely among humans in recent times: H1N1, H2N2, and H3N2. Many humans have acquired at least some level of immunity to these subtypes. All Influenza viruses, however, are known to mutate and change frequently. Influenza viruses are known to infect waterfowl and swine and to circulate among those hosts forming a breeding ground for new subtypes and strains separate from human populations. Because many serotypes (and particularly newly-arising subtypes) have a zero or low prevalence in human populations, there is little or no natural immunity against them in human populations. Such a population is referred to as being "naive" to such serotypes. Accordingly, Influenza viruses might be expected to adapt over time to generate one or more highly virulent strains that will infect and spread catastrophically among naive humans, as has been widely reported in the mainstream press.

The highly-virulent influenza H5N1 subtype (publicly referred to as the bird flu virus), for example, has been reported as having mutated sufficiently to become transmissible from avian hosts to humans. As this subtype has been limited to infecting avian populations in the past, there is little or no legacy of infection to have generated immunity within the human population. Thus, the human population is expected to be highly susceptible to H5N1.

To date, the H5N1 serotype does not appear to have mutated sufficiently to become efficiently transmitted from human to human. Nonetheless, because influenza viruses are constantly adapting, there is concern that H5N1 virus or another virulent influenza strain or serotype will arise that will be able to infect humans and spread easily from one person to another. It has been commonly suggested that if H5N1 virus were to gain the capacity to spread easily from person to person, a worldwide outbreak of disease (i.e., pandemic) would likely begin, resulting in millions of deaths.

Annual influenza outbreaks occur as a result of "antigenic drift." Antigenic drift is caused by mutations within antigenic (i.e., immunity stimulating) portions of viral proteins within viral subtypes circulating in host populations that alter the host's ability to recognize and defend effectively against the infecting virus, even when the virus has been circulating in the community for several years. The antigenic drift that diminishes existing immunity in a host population generally occurs within so-called immunodominant antigens or regions. Immunodominant antigens are those antigens belonging to a pathogen that are the most-easily and most-quickly recognized by the host immune system and, consequently, account for the vast majority of immune response to the invading pathogen. Typically, immunodominant antigens exist within regions of the pathogen that are most exposed to the environment, i.e., are on the external surfaces or on protruding elements of the pathogen, and so are most readily accessible to the host immune system.

In the case of influenza, the immunodominant HA and NA proteins protrude from the central capsid of the viral particle, and so they tend to interact most strongly with the host's internal environment and dominate the host immune response. Mutations occurring in the microbial genome that protect the microbe from the host immune system, these mutations are most readily found to affect the immunodominant antigens.

Conversely, non-immunodominant antigens are those that are capable of raising a host immune response but account for only a small amount of the total immune response. This is thought to happen because the non-immunodominant antigens are at least partially shielded from the host immune system, as in the case of an antigen that is located in a cleft or fold of the microbial surface or is surrounded by protruding elements of the microbe. In the case of influenza, non-immunodominant antigens occurring near the capsid surface are shielded from the host immune system by the immunodominant HA and NA spikes protruding from the surface. Non-immunodominant antigens tend to show less mutation in response to host immune pressure than do immunodominant antigens.

Antigenic shift occurs when there is an abrupt or sudden, major change in a virus. Antigenic shift is typically caused by the occurrence of new combinations of the HA and/or NA proteins on the surface of the virus, i.e., the creation of a new Influenza subtype. The appearance of a new influenza A virus subtype, to which most of the world's population is naive, is the first step toward a pandemic. If the new Influenza subtype also has the capacity to spread easily from person to person, then a full-blown pandemic may be expected resulting in a global influenza outbreak infecting millions of humans.

The CDC and the leading authorities on disease prevention in the world recommend the single best way of preventing the flu is through annual vaccination. Conventional vaccines however, typically target the HA and NA antigens, and have been neither universally protective nor 100 percent effective at preventing the disease. Antigenic shift prevents flu vaccines from being universally protective or from maintaining effectiveness over many years. The ineffectiveness of conventional vaccines may also be due, in part, to antigenic drift and the resulting variation within antigenic portions of the HA and NA proteins most commonly recognized by the immune system (i.e., immunodominant antigens). As a result, many humans may find themselves susceptible to the flu virus without an effective method of treatment available since influenza is constantly improving its resistant to current treatments. This scenario is particularly concerning with respect to the H5N1 virus, which is highly virulent but for which there is currently no widely available commercial vaccine to immunize susceptible human populations.

Currently, flu vaccines are reformulated each year due to the yearly emergence of new strains, and generally induce limited immunity. In addition, to achieve a protective immune response, some vaccines are administered with high doses of antigen. This is particularly true for H5N1 vaccines. In addition, influenza vaccines, including H5N1 vaccines, typically present epitopes in the same order as the epitopes are found in nature, generally presenting as wholeviral proteins; consequently, relatively large amounts of protein are required to make an effective vaccine. As a result, each administration includes an increased cost associated with the dose amount, and there is increased difficulty in manufacturing enough doses to vaccinate the general public. Further, the use of larger proteins elevates the risk of undesirable immune responses in the recipient host.

Accordingly, it would be advantageous to administer a vaccine that provides protection against an infection over a broad range of different strains and/or variations of a pathogen, and a vaccine that is effective against multiple pathogens. It would also be advantageous to administer a single or limited number of vaccinations that would provide effective protection across a selection of different pathogens and a vaccine that could be effective in those individuals with limited immune system function. Such vaccines would be useful to treat many individuals and populations and may be useful to compliment conventional vaccines, all to provide comprehensive protection to as many individuals as possible against existing as well as new and emerging pathogens across a population.

WO2009029686 discloses hybrid polypeptides comprising of influenza antigens and their use as vaccines, including polypeptides containing concatenated "composite" HA and NA epitopes.

### Summary of the Invention

The present invention provides new and useful compositions, as well as tools and methods for generating an immune response. The invention is defined in the claims. In particular, the disclosure provides vaccines and methods developed from multiple antigenic regions of one or more pathogens.

The invention provides a composite antigen comprising SEQ ID NO: 66 or SEQ ID NO: 67. In some embodiments, the composite antigen further comprises epitopes of proteins of Mycobacterium tuberculosis. In some embodiments, the composite antigen comprises SEQ ID NO: 65 or 70. In some embodiments, the composite antigen further comprises epitopes of proteins of Streptococcus, Pseudomonas,Shigella, Campylobacter, Salmonella, Haemophilus influenza, Chlamydophila pneumonia, Corynebacterium diphtheriae, Clostridium tetani, Mycoplasma pneumonia, Staphylococcus aureus, Moraxella catarrhalis, Legionella pneumophila, Bordetella pertussis and Escherichia coli. In some embodiments, the composite antigen comprises SEQ ID NO:94.

The invention also provides a recombinant polynucleotide that encodes the composite antigen of the invention.

The invention also provides the composite antigen of the invention for use in a method for treating or preventing influenza in a mammal, the method comprising administering said composite antigen to the mammal. In some embodiments, the immune response generated is protective against a number of different strains, serotypes or species of the one or more pathogens.

The invention also provides a vaccine comprising the composite antigen of the invention.

The invention also provides a DNA vaccine that encodes the composite antigen of the invention.

One aspect of the disclosure is directed to a composite antigen comprising a peptide with contiguous amino acid sequence derived from a plurality of antigenic epitopes of one or more pathogens that induces an immune response in a mammal that is protective against infection by the one or more pathogens. Preferably the plurality of epitopes contains one or more composite epitopes. Preferably the composite antigen contains a plurality of antigenic epitopes, comprising one or more repetitions of a same epitope, one or more repetitions of different epitopes, one or more repetitions of composite epitopes, or a combination thereof. Also preferably, the amino acid sequence of at least one epitope of the composite antigen does not exist naturally. Composite antigens can be used to treat or preferably prevent infection and disease associated with one or more pathogens including but not limited to viruses, bacteria, parasites, yeast, fungi, or a combination thereof. Preferably the pathogen is an influenza virus and the one or more antigenic epitopes are amino acid sequences of M1, M2, HA, NA, PB1, or PB2 protein, or a combination thereof. Exemplary composite sequences include, but are not limited to, SEQ ID NOs 4, 5, 8, 19, 20, 52, 53, 56 and 54, and SEQ ID NO 16, 65, 66,67, 70 and 73.

Another aspect of the disclosure is directed to composite antigens comprising an amino acid sequence containing amino acids that are in common between sequences of epitopes of multiple conserved regions, and the amino acids that differ between the sequences of epitopes of multiple conserved regions. Preferably, the amino acid sequence has the formula An1BCAn2, wherein A represents the amino acids that are in common between sequences of epitopes of multiple conserved regions, B and C represent the amino acids that differ between the sequences of epitopes of multiple conserved regions, wherein A, B, and C are naturally occurring amino acids, N1 and N2 total to less than 25, and the number of B and C amino acids is less than 3. Exemplary composite sequences include, but are not limited to SEQ ID NO. 6, 7, 21, 22, 54, 55, 58 or 59. Preferably the composite antigen contains multiple conserved regions of a peptide sequence derived from multiple serotypes of a same pathogen. Preferably the pathogen is influenza virus.

Another aspect of the disclosure is directed to an antibody that is specifically reactive to the composite antigen of the disclosure.

Another embodiment of the invention is directed to polynucleotides that encode the composite antigen of the invention.

Another aspect of the disclosure is directed to methods for generating an immune response in a mammal comprising administering to the mammal the composite antigen of the disclosure. Preferably the immune response generated is protective against a number of different strains, serotypes or species of the one or more pathogens.

Another embodiment of the invention is directed to a vaccine comprising the composite antigen of the invention. Preferably the composite antigen is has the formula An1BCAn2, wherein A represents the amino acids that are in common between sequences of epitopes of multiple conserved regions, B and C represent the amino acids that differ between the sequences of epitopes of multiple conserved regions, wherein A, B, and C are naturally occurring amino acids, N1 and N2 total to less than 25, and the number of B and C amino acids is less than 3.

### Description of the Drawings

Figure 1 Summary of ELISA antisera titers of peptides (or Pep) 3, 6, 9, 10 and 11 of H3N2 Influenza virus (Wuhan).
Figure 2 Antisera mean ODs of mice immunized with different peptides of H3N2 Influenza virus (Wuhan 1:40).
Figure 3 Mean ODs of antisera (1:100) on virus and peptide following immunization with Pep 6
Figure 4 Antisera titers for mice immunized with Pep 9 on H1N1 (Caledonia) virus at various dilutions.
Figure 5 Comparisons of fresh (A) and (C) and fixed (B) and (D) binding of Pep 9 on Wuhan coatings (A) and (C) and Caledonia coatings (B) and (D).
Figure 6 Antisera titers from mice immunized with Pep 9 captured on fixed vs. fresh coatings of Wuhan and Caledonia.
Figure 7 Antisera titers for mice immunized with Pep 11 on H5N1 at various dilutions.
Figure 8 Additional sequence information and related information of each sequence (drawings 8-1 to 8-6).

### Description of the Invention

Vaccinations and vaccines are often the best mechanism for avoiding an infection and preventing the spread of debilitating and dangerous pathogens. With respect to viral infections and many bacterial infections, vaccinations are often the only effective option as treatment options are few and those that are available provide only limited effectiveness. Conventional vaccinations require *a priori* understanding or general identification of the existing antigenic regions of the pathogen. The pathogen itself is propagated and a suitable vaccine developed from heat-killed or otherwise attenuated microorganisms. Alternatively, an antigen or collection of antigens is identified that will generate a protective immune response upon administration. The need for a vaccine is especially urgent with respect to preventing infection by certain bacteria and viruses. Many bacteria and especially certain viruses mutate constantly often rendering the vaccine developed to the prior or originating bacteria or virus useless against the new strains that emerge. As a consequence, vaccines against infections are reformulated yearly and often administered at fairly high doses. The manufacturing costs are high and administering vaccines against pose a great many complications and associated risks to patients.

It has been surprisingly discovered that an effective vaccine can be produced from a composite antigen of the invention. Composite antigens are antigens that contains or are derived from a plurality of antigenic regions of a pathogen. The composite antigen of the invention is defined in the claims. Composite antigens of the disclosure may contain one epitope that represents a combination of conserved regions of two or more epitopes (e.g., the composite epitope as outlined herein), or a plurality of immunologically responsive regions derived from one or multiple sources (e.g., virus particles, parasites, bacteria, fungi, cells). These immunological regions are amino acid sequences or epitopes that are representative of sequences found at those antigenic regions of a pathogen or other antigen associated with an infection or a disease or, importantly, associated with stimulation of the immune system to provide protection against the pathogen.

One embodiment of the invention is directed to composite antigens, as defined in the claims. Composite antigens of the invention contain non-naturally occurring amino acid sequences that do not exist in nature and are otherwise artificially constructed. Each sequence of a composite antigen contains a plurality of immunologically responsive regions or epitopes of a pathogen, which are artificially arranged, preferably along a single amino acid sequence. The plurality may contain multiples of the same epitope, although not in a naturally occurring order, or multiples of a variety of different epitopes from one or more pathogens. Epitopes may be identical to known immunological regions of a pathogen, or entirely new constructs that have not previously existed and therefore artificially constructed. Preferably, the composite antigen of the invention induces killer T-cell (T_{C} or CTL) responses, helper T-cell (T_{H}) responses, and specific antibody production in an inoculated mammal.

A "composite" antigen is an engineered, artificially created antigen made from two or more constituent epitopes, such that the resulting composite antigen has physical and/or chemical properties that differ from or are additive of the individual constituent epitopes. Preferable the composite antigen, when exposed to the immune system of a mammal, is capable of simultaneously generating an immunological response to each of the constituent epitope of the composite and preferably to a greater degree (e.g., as measurable from a cellular or humoral response to an identified pathogen) than the individual constituent epitopes. In addition, the composite antigen provides the added function of generating a protective immunological response in a patient when used as a vaccine and against each of the constituent epitopes. Preferably, the composite has the additional function of providing protection against not only the pathogens from which the constituents were derived, but related pathogens as well. These related pathogenic organisms may be strains or serotypes of the same species of organism, or different species of the same genus of organism, or different organisms entirely that are only related by a common epitope.

Another aspect of the disclosure is directed to isolated epitopes. Isolated epitopes are regions obtained or derived from a protein or peptide of a pathogen that elicit a robust immunological response when administered to a mammal. Preferably, that robust response provides the mammal with an immunological protection against developing disease from exposure to the pathogen. A preferred example of an isolated epitope is a composite epitope, which is one artificially created from a combination of two or more highly conserved, although not identical, amino acid sequences of two or more different, but otherwise related pathogens. The pathogens may be of the same type, but of a different strain, serotype, or species or other relation. The composite epitope contains the conserved region that is in common between the related epitopes, and also contains the variable regions which differ. The sequences of a composite epitope that represents a combination of two conserved, but not identical sequences, may be illustrated as follows:

| | |
|---|---|
| Sequence of Epitope 1 | ... AAAAABAAAAA... |
| Sequence of Epitope 2 | ... AAAAACAAAAA... |
| Composite Epitope | ... AAAAABCAAAAA... |

wherein, "A" represents the amino acids in common between the two highly conserved epitopes, "B" and "C" represent the amino acids that differ, respectively, between two epitopes, each of "A",""B" and "C" can be any amino acid and any number of amino acids. Preferably the conserved region contains about 20 or less amino acids on each side of the variable amino acids, preferably about 15 or less, preferably about 10 or less, preferably about 8 or less, preferably about 6 or less, and more preferably about 4 or less. Preferably the amino acids that vary between two similar but not identical conserved regions are 5 or less, preferably 4 or less, preferably 3 or less, preferably 2 or less, and more preferably only 1.

A "composite epitope," similar to the composite antigen, is an engineered, artificially created single epitope made from two or more constituent epitopes, such that the resulting composite epitope has physical and/or chemical properties that differ from or are additive of the constituent epitopes. Preferable the composite epitope, when exposed to the immune system of a mammal, is capable of simultaneously generating an immunological response to each of the constituent epitopes of the composite and preferably to a greater degree than that achieved by either of the constituent epitopes individually. In addition, the composite epitope provides the added function of generating a protective immunological response in a patient when used as a vaccine and against each of the constituent epitopes. Preferably, the composite has the additional function of providing protection against not only the pathogens from which the constituents were derived, but related pathogens as well. These related pathogenic organisms may be strains or serotypes of the same species of organism, or different species of the same genus of organism, or different organisms entirely that are only related by a common epitope.

Composite epitopes of the invention are entirely artificial molecules that do not otherwise exist in nature and to which an immune system has not been otherwise exposed. Preferably, these conserved immunological regions that are combined as a composite epitope represent immunologically responsive regions of proteins and/or polypeptides that are highly conserved between related pathogens. Although a vaccine can be developed from a single composite epitope, in many instances the most effective vaccine may be developed from multiple, different composite epitopes.

Accordingly, composite antigens of the disclosure may contain one or more composite epitopes and/or one or more known epitopes to provide an effective vaccine. Although composite antigens may comprise a single composite epitope, a composite antigen would not comprise only a single known epitope. Preferably, the immunological response achieved from a vaccination with a composite antigen, or group of composite antigens, provides protection against infection caused by the original strains from which the sequence of the composite antigen was derived, and also provides immunological protection against other strains, serotypes and/or species that share one or more of the general conserved regions represented in the composite antigen. Thus, the resulting immune response achieved from a vaccination with a composite antigen is more broadly protective than can be achieved from a conventional single antigen vaccination against multiple strains, serotypes, and species or otherwise related pathogens regardless of antigenic drift that may take place in the evolution of the pathogen. Preferably, vaccines developed from composite antigens of the invention avoid any need for repeated or annual vaccinations, the associated complications and expenses of manufacture, and the elevated risks to the patient. These vaccines are useful to treat individuals and populations, thereby preventing infection, mortality and pandemics, and are useful to compliment conventional vaccines.

As discussed herein, the composite antigen preferably comprises a single chain of amino acids with a sequences derived from one or more composite eptiopes, one or more composite epitopes plus one or more known epitopes, or a plurality of known epitopes, that may be the same or different. Epitope sequences may be repeated consecutively and uninterrupted along a composite sequence or interspersed among other sequences that may be single or a few amino acids as spacers or sequences that encode peptides (collectively spacers), and may be nonimmunogenic or immunogenic and capable of inducing a cellular (T cell) or humoral (B cell) immune response in a mammal. Peptides sequence from unrelated microbes may be combined into a single composite antigen. For example, viral sequences of selected immunoresponsive peptides may be interspersed with conserved sequences or epitopes selected from other microbes, such as, for example, bacteria such as *S. pneumococcus, P. auriginosa* or *S. aureus.* Preferred viral proteins, from which preferred epitopes may be selected, include, but are not limited to the influenza virus proteins PspA, PspC, HA, NA, M2e, H. influenza protein D, and coagulase.

An epitope of the composite antigen may be of any sequence and size, but is preferable composed of natural amino acids and is more than 5 but less than 35 amino acids in length, preferably less than 30, preferably between 5 and 25 amino acids in length, preferably between 8 and 20 amino acids in length, and more preferably between 5 and 15 amino acids in length. Composite antigens preferably contain any number of composite and/or other epitopes. The most effective number of epitopes of a composite antigen against a particular pathogen, pathogen family, or group of pathogens may be determined by one skilled in the art from the disclosures of this application and using routine testing procedures. Composite antigens may be effective with one epitope, preferably with 2 or more, 3 or more 4 or more, 5 or more or greater. Optionally, composite antigens may include one or more spacers between epitopes which may be sequences of antigenic regions derived from the same or from one or more different pathogens, or sequences that serve as immunological primers or that otherwise provide a boost to the immune system. That boost may be generated from a sequence of amino acids that are known to stimulate the immune system, either directly or as an adjuvant. In one preferred embodiment, composite antigens useful to generate an immunological response against influenza virus comprise epitopes of HA and/or NA proteins, and/or new epitopes derived from similar conserved regions of different serotypes of influenza virus. Also preferred are composite antigens containing epitopes of proteins of *Mycobacterium tuberculosis* and *Clostridium tetani,* and/or new epitopes derived from similar conserved regions of different serotypes of these bacteria.

Another aspect of the disclosure is directed to a contiguous sequence of one or more epitopes, which may comprise composite and/or known epitopes, from one or more pathogens in a sequence that does not exist naturally and must be artificially constructed. Preferably, a contiguous sequence of the disclosure contains one or more composite epitopes, which is a combination of the sequences of the conserved regions of epitopes that are common to multiple pathogens plus those amino acids that differ between the two conserved regions. For example, where two pathogens contain similar conserved regions that differ by only a single amino acid, the composite sequences would include the conserved region amino acids and each of the amino acids that differ between the two regions as discussed herein.

It is also preferable that a composite antigen of the disclosure contain a plurality of repeated epitopes and, optionally, epitopes conjugated with linker regions between or surrounding each epitope, and the plurality of epitopes be the same or different. Preferred linkers include amino acid sequences of antigenic regions of the same or of different pathogens, or amino acids sequences that aid in the generation of an immune response. Preferred examples include, but are not limited to any of the various antigenic regions of bacteria such as, but not limited to tuberculosis and virus such as, but not limited to influenza. It is also preferred that composite antigens contain epitopes that generate a T cell response, a B cell response, or both in conjunction with a specific response to the pathogen.

Another aspect of the disclosure is directed to immunizing animals with the composite antigens of the disclosure. Antisera obtained from the immunized animals are reactive against the pathogens from which the composite antigen was derived. Another aspect of the disclosure is therefore antisera obtained from the immunized animals, which may be further purified for testing or utilized therapeutically for administration to another mammal and thereby provides protection against infection from the one or more pathogens. It is also preferred that the antisera obtained provide a broad protection, not just against the pathogens from which the composite antigen was derived, but also from additional pathogens that may differ by strain, serotype, or even species.

Another aspect of the disclosure is a vaccine composed of the composite antigen or antisera developed from the composite antigen of the disclosure. Preferably, the vaccines of the invention are less susceptible to variation of antigenicity due to antigenic shift of pathogens which reduces or eliminates the need for annual or repeated vaccination to maintain protection of patient populations against potential outbreaks of infection from new viral isolates. In addition, the vaccines of the invention generally and advantageously provide increased safety considerations, both in their manufacture and administration (due in part to a substantially decreased need for repeated administration), a relatively long shelf life in part due to minimized need to reformulate due to strain-specific shift and drift, an ability to target immune responses with high specificity for particular microbial epitopes, and an ability to prepare a single vaccine that is effective against multiple pathogens, each of which may be a different but know strain or species of the same pathogen. The disclosure encompasses antigenic and antibody compositions, methods of making such compositions, and methods for their use in the prevention, treatment, management, and/or prophylaxis of an infection. The compositions disclosed herein, as well as methods employing them, find particular use in the treatment or prevention of viral, bacterial, parasitic and/or fungal pathogenesis and infection using immunogenic compositions and methods superior to conventional treatments presently available in the art.

Another aspect of the disclosure is directed to methods of preventing or controlling infection, such as, for example, an outbreak of viral, parasitic, fungal or bacterial infection, preferably but not limited to an influenza virus and/or a tuberculosis bacterial infection, in a selected mammalian population. The method includes at least the step of providing an immunologically effective amount of one or more of the disclosed immunogenic or vaccine compositions to a susceptible or an at-risk member of the population, for a time sufficient to prevent, reduce, lessen, alleviate, control, or delay the outbreak of such an infection in the general population.

Another aspect of the disclosure is directed to methods for producing a protective immune response against infection, for example by influenza virus, in a mammal in need thereof. Such a method generally includes a step of providing to a mammal in need thereof, an immunologically-effective amount of one or more of the immunogenic compositions disclosed herein under conditions and for a time sufficient to produce such a protective immune response against one or more species, strains, or serotypes of an infectious organism. Additionally, the disclosure also provides a method for administering a prophylactic antiviral or antimicrobial composition to at least a first cell, tissue, organ, or organ system in a mammal that generally involves providing to such a mammal a prophylactically-effective amount of at least a first immunogenic composition as disclosed herein.

Another aspect of the disclosure is directed to an immunogenic composition comprising the composite antigens of the disclosure having one or more repeated peptide sequences, or fragments, variants, or derivatives of such peptide sequences that are conserved across a plurality of proteins in the same or different pathogen. The conserved regions from which the composite sequence is derived may be conserved within subtypes of the same pathogen or different pathogens. Preferred pathogens include, but are not limited to bacteria, viruses, parasites, fungi and viruses.

Composite antigens of the disclosure may also be obtained or derived from the sequences of bacteria such as, for example, multiple or combined epitopes of the proteins and/or polypeptides of, for example, but not limited to *Streptococcus, Pseudomonas, Mycobacterium such as M. tuberculosis, Shigella, Campylobacter, Salmonella, Haemophilus influenza, Chlamydophila pneumonia, Corynebacterium diphtheriae, Clostridium tetani, Mycoplasma pneumonia, Staphylococcus aureus, Moraxella catarrhalis, Legionella pneumophila, Bordetella pertussis, Escherichia coli,* such as *E. coli 0157,* and multiple or combined epitomes of conserved regions of any of the foregoing. Exemplary parasites from which sequences may be obtained or derived include but are not limited to *Plasmodium* such as *Plasmodium falciparum* and *Trypanosoma.* Exemplary fungi include, but are not limited to *Aspergillus fumigatus* or *Aspergillus flavus.* Exemplary viruses include, but are not limited to arena viruses, bunyaviruses, coronaviruses, filoviruses, hepadna viruses, herpes viruses, orthomyxoviruses, parvoviruses, picornaviruses, papillomaviruses, reoviruses, retroviruses, rhabdoviruses, and togaviruses. Preferably, the virus epitopes are obtained or derived from sequences of Influenza viruses (e.g., the paramyxoviruses).

In another preferred embodiment, the composite antigens contain a conserved region derived from an influenza virus subtypes (e.g., influenza viruses with varying HA or NA compositions, such as H1N1, H5N1, H3N2, and H2N2). Epitopes of conserved regions on NA or HA may also confer cross-subtype immunity. As an example, conserved epitopes on NA(N1) may confer enhanced immunity to H5N1 and H1N1. With respect to similar or homologous chemical compounds among influenza A subtypes and/or strains within a subtype, preferably these are at least about 80 percent, more preferably at least about 90 percent, more preferably at least about 95 percent identical, more preferably at least about 96 percent identical, more preferably at least about 97 percent identical, more preferably at least about 98 percent identical, more preferably at least about 99 percent identical, and even more preferably 100 percent identical (invariant). Preferably, at least one peptide sequence within the composite antigen is also conserved on homologous proteins (e.g., protein subunits) of at least two viral particles, preferably influenza particles. Proteins of influenza virus include, for example, expressed proteins in the virus structure, such as HA, NA, protein polymerases (PB1, PB2, PA), matrix proteins (M1, M2), and nucleoprotein ("NP"). Preferably, the conserved peptide sequences are conserved on at least two or more of the M1, M2, HA, NA, or one or more polymerase proteins.

In a preferred example, a selected sequence in the M1 and M2 proteins of the H5N1 influenza virus corresponds to the M1 and M2 proteins found in other H5N1 particles, and to the same sequence in the M1 and M2 proteins of the H3N2 influenza virus. In addition, while HA and NA proteins have highly variable regions, conserved sequences from HA and NA are found across many influenza strains and many subtypes (e.g., HA and NA sequences are conserved across H5N1 and H1N1). In a preferred aspect of the disclsoure, the composite sequences is derived from a conserved sequence present within variants or strains (viral isolates expressing substantially the same HA and NA proteins, but wherein the HA and NA protein amino acid sequences show some minor drift), of a single influenzavirus subtype and more preferably across at least two influenzavirus subtypes, e.g., subtypes of influenza A virus.

In another aspect, the disclosure provides a composite peptide or polypeptide that includes at least one epitopic antigen, which comprises one or more repeatedly occurring epitope sequences, each of which is conserved across a plurality of homologous proteins that is conserved in a population of influenzavirus strains or serotypes, and a pharmaceutically acceptable carrier. In exemplary composite antigens, at least one epitopic sequence is repeated at least once, preferably at least twice times, more preferably at least three times. In other aspects of the disclosure, the at least one epitopic sequence is repeated four or more times. Preferably, the composite sequences are identical with the sequences in the homologous protein subunits of at least two circulating viral isolates. In each aspect, the compositions may include a pharmaceutically acceptable carrier.

In additional preferred aspect, the composite peptide sequences include sequences derived from genome (i.e., RNA) segment 7 of the influenza virus, while in a more preferred aspects of the disclosure, the sequences include at least portions of the M1 and M2 proteins. In other preferred aspects of the disclosure, the composite sequences include sequences expressed from genome segments encoding the HA or NA proteins. Such sequences are less affected by subtype drift and more broadly protective against infections.

In additional aspects of the disclosure, the composite antigen includes one or more T-cell stimulating epitopes, such as diphtheria toxoid, tetanus toxoid, a polysaccharide, a lipoprotein, or a derivative or any combination thereof (including fragments or variants thereof). Typically, the at least one repeated sequence of the composite antigen is contained within the same molecule as the T-cell stimulating epitopes. In the case of protein-based T-cell stimulating epitopes, the at least one repeated sequence of the composite antigen may be contained within the same polypeptide as the T-cell stimulating epitopes, may be conjugated thereto, or may be associated in other ways. Preferably, at least one repeated sequence is incorporated within or alongside the one or more T-cell stimulating epitopes in a composite antigen of the disclosure.

In additional aspects of the disclosure, the composite antigens, with or without associated T-cell stimulating epitopes may include one or more polysaccharides or portions thereof. In preferred aspects of the disclosure, at least one sequence of a composite antigen is conjugated to one or more polysaccharides. In other aspects of the disclosure, one or more polysaccharides are conjugated to other portions of the composite antigen. Certain aspects of the present disclosure are selected from polysaccharide vaccines, protein-polysaccharide conjugate vaccines, or combinations thereof.

Composite antigens of the invention may be synthesizing by in vitro chemical synthesis, solid-phase protein synthesis, and in vitro (cell-free) protein translation, or recombinantly engineered and expressed in bacterial cells, fungi, insect cells, mammalian cells, virus particles, yeast, and the like.

A preferred composite antigen includes at least one of the following elements: at least one repeated composite sequence; at least one T-cell epitope; at least one polysaccharide; at least one polynucleotide; at least one structural component; or a combination thereof. The at least one structural component may include one or more of: at least one linker segment; at least one sugarbinding moiety; at least one nucleotide-binding moiety; at least one protein-binding moiety; at least one enzymatic moiety; or a combination thereof. The disclosure encompasses methods of preparing an immunogenic composition, preferably a pharmaceutical composition, more preferably a vaccine, wherein a target antigen of the present disclosure is associated with a pharmaceutically acceptable diluent, excipient, or carrier, and may be used with most any adjuvant.

A relatively small number of conservative or neutral substitutions (e.g., 1 or 2) may be made within the sequence of the composite antigen or epitope sequences disclosed herein, without substantially altering the immunological response to the peptide. In some cases, the substitution of one or more amino acids in a particular peptide may in fact serve to enhance or otherwise improve the ability of the peptide to elicit an immune or T-cell response in an animal that has been provided with a composition that comprises the modified peptide, or a polynucleotide that encodes the peptide. Suitable substitutions may generally be identified using computer programs and the effect of such substitutions may be confirmed based on the reactivity of the modified peptide with antisera and/or T-cells. Accordingly, within certain preferred aspects of the disclosure, a peptide for use in the disclosed diagnostic and therapeutic methods may comprise a primary amino acid sequence in which one or more amino acid residues are substituted by one or more replacement amino acids, such that the ability of the modified peptide to react with antigen-specific antisera and/or T-cell lines or clones is not significantly less than that for the unmodified peptide.

As described above, preferred peptide variants are those that contain one or more conservative substitutions. A "conservative substitution" is one in which an amino acid is substituted for another amino acid that has similar properties, such that one skilled in the art of peptide chemistry would expect the secondary structure and hydropathic nature of the peptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Examples of amino acid substitutions that represent a conservative change include: (1) replacement of one or more Ala, Pro, Gly, Glu, Asp, Gln, Asn, Ser, or Thr; residues with one or more residues from the same group; (2) replacement of one or more Cys, Ser, Tyr, or Thr residues with one or more residues from the same group; (3) replacement of one or more Val, Ile, Leu, Met, Ala, or Phe residues with one or more residues from the same group; (4) replacement of one or more Lys, Arg, or His residues with one or more residues from the same group; and (5) replacement of one or more Phe, Tyr, Trp, or His residues with one or more residues from the same group. A variant may also, or alternatively, contain non-conservative changes, for example, by substituting one of the amino acid residues from group (1) with an amino acid residue from group (2), group (3), group (4), or group (5). Variants may also (or alternatively) be modified by, for example, the deletion or addition of amino acids that have minimal influence on the immunogenicity, secondary structure and hydropathic nature of the peptide.

Epitopes may be arranged in any order relative to one another in the composite sequence. The number of spacer amino acids between two or more of the epitopic sequences can be of any practical range, including, for example, from 1 or 2 amino acids to 3, 4, 5, 6, 7, 8, 9, or even 10 or more amino acids between adjacent epitopes.

Another aspect of the disclosure is directed to polynucleotides including DNA, RNA and PNA constructs that encode the composite sequences of the disclosure. These polynucleotides may be single-stranded (coding or antisense) or double-stranded, and may be DNA (genomic, cDNA or synthetic) or RNA molecules. Additional coding or non-coding sequences may, but need not, be present within a polynucleotide of the present disclosure, and a polynucleotide may, but need not, be linked to other molecules and/or support materials. As is appreciated by those of ordinary skill in the art that, as a result of the degeneracy of the genetic code, there are many nucleotide sequences that encode a given primary amino acid sequence. Some of these polynucleotides bear minimal homology to the nucleotide sequence of any native gene. Nonetheless, polynucleotides that vary due to differences in codon usage are specifically contemplated by the present disclosure. Polynucleotides that encode an immunogenic peptide may generally be used for production of the peptide, in vitro or in vivo. Any polynucleotide may be further modified to increase stability in vivo. Possible modifications include, but are not limited to, the addition of flanking sequences at the 5' and/or 3'-ends; the use of phosphorothioate or 2'-o-methyl rather than phosphodiesterase linkages in the backbone; and/or the inclusion of nontraditional bases such as inosine, queosine and wybutosine, as well as acetyl- methyl-, thioand other modified forms of adenine, cytidine, guanine, thymine and uridine.

Another aspect of the disclosure encompasses methods of vaccinating a subject against Influenza that includes administering to a patient in need of influenza vaccination a therapeutically or prophylactically effective amount of an influenza vaccine, which influenza vaccine includes a composite antigen comprising one or more repeatedly occurring composite or other sequences, each of which is conserved across a plurality of homologous proteins in a plurality of influenza virus particles, and a pharmaceutically acceptable carrier, to provide a detectable immune response in the patient against influenza.

Another embodiment of the invention is directed to nucleotide or DNA vaccines encoding the composite antigens of the invention. A DNA vaccine of the invention contains the genetic sequence of a composite antigen, plus other necessary sequences that provide for the expression of the composite antigen in cells. By injecting the mammal with the genetically engineered DNA, the composite antigen is produced in or preferably on cells, which the mammal's immune system recognizes and thereby generates a humoral or cellular response to the composite antigen, and therefore the pathogen. DNA vaccines have a number of advantages over conventional vaccines, including the ability to induce a more general and complete immune response in the mammal. Accordingly, DNA vaccines can be used to protect a mammal against disease caused from many different pathogenic organisms of viral, bacterial, and parasitic origin as well as certain tumors.

DNA vaccines typically comprise a bacterial DNA contained that encodes the composite antigen contained in vectors or plasmids that have been genetically modified to transcribe and translate the composite antigenic sequences into specific protein sequences derived from a pathogen. By way of example, the vaccine DNA is injected into the cells of the body, where the cellular machinery transcribed and translates the DNA into the composite antigen. Composite antigens, being non-natural and unrecognized by the mammalian immune system, are processed by cells and the processed proteins, preferably the epitopes, displayed on cell surfaces. Upon recognition of these composite antigens as foreign, the mammal's immune system generates an appropriate immune response that protects the mammal from infection. In addition, DNA vaccine of the invention are preferably codon optimized for expression in the mammalian cells of interest, such as but not limited to mouse or human cells. In a preferred embodiment, codon optimization involves selecting a desired codon usage bias (the frequency of occurrence of synonymous codons in coding DNA) for the particular cell type so that the desired peptide sequence is expressed.

Another aspect of the disclosure is directed to therapeutic and prophylactic agents in a pharmaceutically acceptable composition for administration to a cell or an animal, either alone, or in combination with one or more other modalities of prophylaxis and/or therapy. Therapeutic and prophylactic agents of the disclosure include composite antigens, composite epitopes, compositions containing composite antigens and epitopes, composite sequences, DNA vaccines of the disclosure, antibodies of the disclosure, and/or T cells primed or exposed to composite antigens of the disclosure. The formulation of pharmaceutically-acceptable excipients and carrier solutions is well known to those of ordinary skill in the art, as is the development of suitable dosing and treatment regimens for using the particular compositions described herein in a variety of treatment regimens.

The amount of immunogenic composition(s) and the time needed for the administration of such immunogenic composition(s) will be within the purview of the ordinary-skilled artisan having benefit of the present teachings. The administration of a therapeutically-effective, pharmaceutically-effective, and/or prophylactically-effective amount of the disclosed immunogenic compositions may be achieved by a single administration, such as for example, a single injection of a sufficient quantity of the delivered agent to provide the desired benefit to the patient undergoing such a procedure. Alternatively, in some circumstances, it may be desirable to provide multiple, or successive administrations of the immunogenic compositions, either over a relatively short, or even a relatively prolonged period of time, as may be determined by the medical practitioner overseeing the administration of such compositions to the selected individual.

The immunogenic compositions and vaccines of the present disclosure are preferably administered in a manner compatible with the dosage formulation, and in such an amount as will be prophylactically or therapeutically effective and preferably immunogenic. The quantity to be administered depends on the subject to be treated, including, e.g., the capacity of the patient's immune system to mount an immune response, and the degree of protection desired. Suitable dosage ranges may be on the order of several hundred micrograms (µg) of active ingredient per vaccination with a preferred range from about 0.1 µg to 2000 µg (even though higher amounts, such as, e.g., in the range of about 1 to about 10 mg are also contemplated), such as in the range from about 0.5 µg to 1000 µg, preferably in the range from about 1 µg to about 500 µg and especially in the range from about 10 µg to about 100 µg. Suitable regimens for initial administration and booster shots are also variable but are typified by an initial administration followed by optional but preferred subsequent inoculations or other periodic administrations.

In certain embodiments, the dose would consist of the range of about 1 µg to about 1 mg total protein or target antigen. In one exemplary embodiment, the vaccine dosage range is about 0.1 µg to about 10 mg. However, one may prefer to adjust dosage based on the amount of peptide delivered. In either case, these ranges are merely guidelines from which one of ordinary skill in the art may deviate according to conventional dosing techniques. Precise dosages may be determined by assessing the immunogenicity of the conjugate produced in the appropriate host so that an immunologically effective dose is delivered. An immunologically effective dose is one that stimulates the immune system of the patient to establish an immune response to the immunogenic composition or vaccine. Preferably, a level of immunological memory sufficient to provide long-term protection against disease caused by microbial infection is obtained. The immunogenic compositions or vaccines of the invention may be preferably formulated with an adjuvant. By "long-term" it is preferably meant over a period of time of at least about 6 months, over at least about 1 year, over at least about 2 to 5 or even at least about 2 to about 10 years or longer.

Another aspect of the disclosure is directed to antibodies that are specific for the composite antigens as described here and conservative variants thereof. Antibodies specific for these polypeptides are useful, e.g., in both diagnostic and therapeutic purposes, e.g., related to the activity, distribution, and expression of target polypeptides. Antibodies of the disclosure may be classes IgG, IgM, IgA, IgD or IgE and include, but are not limited to, polyclonal antibodies, monoclonal antibodies, multiple or single chain antibodies, including single chain Fv (sFv or scFv) antibodies in which a variable heavy and a variable light chain are joined together (directly or through a peptide linker) to form a continuous polypeptide, and humanized or chimeric antibodies.

Antibodies specific for the composite peptides of the disclosure can be generated by methods well known in the art. Such antibodies can include, but are not limited to, polyclonal, monoclonal, chimeric, humanized, single chain, Fab fragments and fragments produced by an Fab expression library. Numerous methods for producing polyclonal and monoclonal antibodies are known to those of skill in the art, and can be adapted to produce antibodies specific for the polypeptides of the disclosure, and/or encoded by the polynucleotide sequences of the disclosure (see, e.g., Coligan Current Protocols in Immunology Wiley/Greene, NY; Paul (ed.) (1991); (1998) Fundamental Immunology Fourth Edition, Lippincott-Raven, Lippincott Williams & Wilkins; Harlow and Lane (1989) Antibodies: A Laboratory Manual, Cold Spring Harbor Press, NY, USA; Stites et al. (Eds.) Basic and Clinical Immunology (4th ed.) Lange Medical Publications, Los Altos, CA, USA and references cited therein; Goding, Monoclonal Antibodies: Principles and Practice (2d ed.) Academic Press, New York, NY, USA; 1986; and Kohler and Milstein (1975).

### Examples

Any examples falling outside the scope of the claims is provide for comparative purposes only. Sequences

The following is a list of exemplary sequence. These sequences include composite sequences as well as sequences of interest that can be combined to form composite sequences of the disclosure:

| | |
|---|---|
| SEQ ID NO 1 | DWSGYSGSFVQHPELTGLD (N1 sequence; H1 N5 |
| SEQ ID NO 2 | ETPIRNE (N1 epitope) |
| SEQ ID NO 3 | FVIREPFISCSHLEC (Pep 5) |
| SEQ ID NO 4 | GNFIAP (HA epitope; Pep 1) |
| SEQ ID NO 5 | GNLIAP (HA epitope; Pep 2) |
| SEQ ID NO 6 | GNLFIAP (composite sequence of SEQ ID NOs 4 and 5; Pep 3) |
| SEQ ID NO 7 | GNLIFAP (composite sequence of SEQ ID NOs 4 and 5) |
| SEQ ID NO 8 | HYEECSCY (NA epitope; Pep 10) |
| SEQ ID NO 9 | LLTEVETPIR |
| SEQ ID NO 10 | LLTEVETPIRN |
| SEQ ID NO 11 | LLTEVETPIRNE |
| SEQ ID NO 12 | DWSGYSGSFVQHPELTGL (N1 sequence; H1 N5) |
| SEQ ID NO 13 | EVETPIRNE |
| SEQ ID NO 14 | FLLPEDETPIRNEWGLLTDDETPIRYIKANSKFIGITE |
| SEQ ID NO 15 | GNLFIAPGNLFIAPHYEECSCYHYEECSCYQYIKANSKFIGITEHY EECSCYTPIRNETPIRNE |
| SEQ ID NO 16 | GNLFIAPGNLFIAPQYIKANSKFIGITEGNLFIAP (composite of SEQ ID NO 6, SEQ ID NO 6, SEQ ID NO 60, and SEQ ID NO 6) |
| SEQ ID NO 17 | HYEECSCYDWSGYSGSFVQHPELTGLHYEECSCYQYIKAN SKFIGITE |
| SEQ ID NO 18 | ITGFAPFSKDNSIRLSAGGDIWVTREPYVSCDP |
| SEQ ID NO 19 | IWGIHHP (HA epitope) |
| SEQ ID NO 20 | IWGVHHP (HA epitope) |
| SEQ ID NO 21 | IWGVIHHP (composite of SEQ ID NOs. 19 and 20) |
| SEQ ID NO 22 | IWGIVHHP (composite of SEQ ID NOs. 19 and 20) |
| SEQ ID NO 23 | KSCINRCFYVELIRGR |
| SEQ ID NO 24 | LLTEVETPIRNESLLTEVETPIRNEWG (M2e epitope) |
| SEQ ID NO 25 | LLTEVETPIRNEW (M2e epitope) |
| SEQ ID NO 26 | LLTEVETPIRNEWG (M2e epitope) |
| SEQ ID NO 27 | LTEVETPIRNE (M2e epitope) |
| SEQ ID NO 28 | LTEVETPIRNEW (M2e epitope) |
| SEQ ID NO 29 | LTEVETPIRNEWG (M2e epitope) |
| SEQ ID NO 30 | MSLLTEVET (M2e epitope) |
| SEQ ID NO 31 | MSLLTEVETP (M2e epitope) |
| SEQ ID NO 32 | MSLLTEVETPI (M2e epitope) |
| SEQ ID NO 33 | MSLLTEVETPIR (M2e epitope) |
| SEQ ID NO 34 | MSLLTEVETPIRN (M2e epitope) |
| SEQ ID NO 35 | MSLLTEVETPIRNE (M2e epitopes) |
| SEQ ID NO 36 | MSLLTEVETPIRNETPIRNE (M2e epitope) |
| SEQ ID NO 37 | MSLLTEVETPIRNEW (M2e epitope) |
| SEQ ID NO 38 | MSLLTEVETPIRNEWG (M2e epitope) |
| SEQ ID NO 39 | MSLLTEVETPIRNEWGCRCNDSSD (M2e epitope) |
| SEQ ID NO 40 | SLLTEVET (M2e epitope) |
| SEQ ID NO 41 | SLLTEVETPIRNE (M2e epitope) |
| SEQ ID NO 42 | SLLTEVETPIRNEW (M2e epitope) |
| SEQ ID NO 43 | SLLTEVETPIRNEWG (M2e epitope) |
| SEQ ID NO 44 | SLLTEVETPIRNEWGTPIRNE (M2e epitope) |
| SEQ ID NO 45 | SLLTEVETPIRNEWGTPIRNETPIRNE (M2e epitope) |
| SEQ ID NO 46 | SLLTEVETPIRNEWGTPIRNETPIRNETPIRNE (M2e epitopes) |
| SEQ ID NO 47 | SLLTEVETPIRNEWGLLTEVETPIRQYIKANSKFIGITE (M2e epitope) |
| SEQ ID NO 48 | TEVETPIRNE (M2e epitope) |
| SEQ ID NO 49 | TPIRNE |
| SEQ ID NO 50 | VETPIRNE |
| SEQ ID NO 51 | VTREPYVSCDPKSCINRCFYVELIRGRVTREPYVSCDPWYIK ANSKFIGITE |
| SEQ ID NO 52 | WGIHHP (HA conserved region; Pep 5) |
| SEQ ID NO 53 | WGVHHP (HA conserved region; Pep 4) |
| SEQ ID NO 54 | WGVIHHP (composite of SEQ ID NOs 52 and 53; Pep 6) |
| SEQ ID NO 55 | WGIVIH-IP (composite of SEQ ID NOs 52 and 53; Pep 7) |
| SEQ ID NO 56 | YIWGIHHP |
| SEQ ID NO 57 | YIWGVHHP |
| SEQ ID NO 58 | YIWGVIHHP (composite of SEQ ID NOs 56 and 57) |
| SEQ ID NO 59 | YIWGIVHHP (composite of SEQ ID NOs 56 and 57) |
| SEQ ID NO 60 | QYIKANSKFIGITE |
| SEQ ID NO 61 | PIRNEWGCRCNDSSD |

SEQ ID NO 65
SEYAYGSFVRTVSLPVGADEGNLFIAPWGVIHHPHYEECSCYGLPVEYLQVPSPSMGRDI
KVQFQSGGANSPALYLLDGLRAQDDFSGWDINTPAFEWYDQSGLSVVMPVGGQSSFYS
DWYQPACRKAGCQTYKWETFLTSELPGWLQANRHVQPTGSAVVGLSMAASSALTLAI
YHPQQFVYAGAMSGLLDPSQAMGPTLIGLAMGDAGGYKASDMWGPKEDPAWQRNDP
LLNVGKLIANNTRVWVYCGNGKPSDLGGNNLPAKFLEGFVRTSNIKFQDAYNAGGHNG
VFDFPDSGTHSWEYWGAQLNAMKPDLQRHWVPRPTPGPPQGAFDFPDSGTHSWEYWG
AQLNAMKPDLQRHWVPRPTPGPPQGA (Sequence for DNA vaccine development 373
amino acids; has a TB conserved regions on each side of Pep 11)

| | |
|---|---|
| SEQ ID NO 66 | GNLFIAPWGVIHHPHYEECSCY (SEQ ID NOs 6, 54, and 8; Pep 11) |
| SEQ ID NO 67 | WGVIHHPGNLFIAPHYEECSCY (SEQ ID NOs 54, 6, and 8) |

SEQ ID NO 68 
SRPGLPVEYLQVPSPSMGRDIKVQFQSGGANSPALYLLDGLRAQDDFSGWDINTPAFEW
YDQSGLSVVMPVGGQSSFYSDWYQPACGKAGCQTYKWETFLTSELPGWLQANRHVKP
TGSAVVGLSMAASSALTLAIYHPQQFVYAGAMSGLLDPSQAMGPTLIGLAMGDAGGY
KASDMWGPKEDPAWQRNDPLLNVGKLIANNTRVWVYCGNGKPSDLGGNNLPAKFLE
GFVRTSNIKFQDAYNAGGGHNGVFDFPDSGTHSWEYWGAQLNAMKPDLQRALGATPN
TGPAPQGA (TB coding region sequence of 85a)
SEQ ID NO 69 
TCCCGGCCGGGCTTGCCGGTGGAGTACCTGCAGGTGCCGTCGCCGTCGATGGGCCGT
GACATCAAGGTCCAATTCCAAAGTGGTGGTGCCAACTCGCCCGCCCTGTACCTGCTC
GACGGCCTGCGCGCGCAGGACGACTTCAGCGGCTGGGACATCAACACCCCGGCGTT
CGAGTGGTACGACCAGTCGGGCCTGTCGGTGGTCATGCCGGTGGGTGGCCAGTCAA
GCTTCTACTCCGACTGGTACCAGCCCGCCTGCGGCAAGGCCGGTTGCCAGACTTACA
AGTGGGAGACCTTCCTGACCAGCGAGCTGCCGGGGTGGCTGCAGGCCAACAGGCAC
GTCAAGCCCACCGGAAGCGCCGTCGTCGGTCTTTCGATGGCTGCTTCTTCGGCGCTG
ACGCTGGCGATCTATCACCCCCAGCAGTTCGTCTACGCGGGAGCGATGTCGGGCCTG
 
TTGGACCCCTCCCAGGCGATGGGTCCCACCCTGATCGGCCTGGCGATGGGTGACGCT
GGCGGCTACAAGGCCTCCGACATGTGGGGCCCGAAGGAGGACCCGGCGTGGCAGCG
CAACGACCCGCTGTTGAACGTCGGGAAGCTGATCGCCAACAACACCCGCGTCTGGG
TGTACTGCGGCAACGGCAAGCCGTCGGATCTGGGTGGCAACAACCTGCCGGCCAAG
TTCCTCGAGGGCTTCGTGCGGACCAGCAACATCAAGTTCCAAGACGCCTACAACGCC
GGTGGCGGCCACAACGGCGTGTTCGACTTCCCGGACAGCGGTACGCACAGCTGGGA
GTACTGGGGCGCGCAGCTCAACGCTATGAAGCCCGACCTGCAACGGGCACTGGGTG
CCACGCCCAACACCGGGCCCGCGCCCCAGGGCGCC (nucleotide sequence corresponding
to TB sequence 85a or SEQ ID NO 64)
SEQ ID NO 70 
SEFAYGSFVRTVSLPVGADEGNLFIAPWGVIHHPHYEECSCYSRPGLPVEYLQVPSPSMG
RDIKVQFQSGGANSPALYLLDGLRAQDDFSGWDINTPAFEWYDQSGLSVVMPVGGQSS
FYSDWYQPACGKAGCQTYKWETFLTSELPGWLQANRHVKPTGSAVVGLSMAASSALT
LAIYHPQQFVYAGAMSGLLDPSQAMGPTLIGLAMGDAGGYKASDMWGPKEDPAWQR
NDPLLNVGKLIANNTRVWVYCGNGKPSDLGGNNLPAKFLEGFVRTSNIKFQDAYNAGG
GHNGVFDFPDSGTHSWEYWGAQLNAMKPDLQRALGATPNTGPAPQGA (336 amino acid
sequence comprising HSPx, Pep 11 and TB 85a)
SEQ ID NO 71

TTTGGGCCCATTATGTCGGAATTCGCGTACGGTTCCTTCGTTCGCACGGTGTCGCTGC
CGGTAGGTGCTGACGAGGGGAATCTAttcATTGCTCCTTGGGGGGTTattCA
CCACCCGCATTATGAGGAATGTTCCTGTTACTCCCGGCCGGGCTTGCCGG
TGGAGTACCTGCAGGTGCCGTCGCCGTCGATGGGCCGTGACATCAAGGTCCAATTCC
AAAGTGGTGGTGCCAACTCGCCCGCCCTGTACCTGCTCGACGGCCTGCGCGCGCAG
GACGACTTCAGCGGCTGGGACATCAACACCCCGGCGTTCGAGTGGTACGACCAGTC
GGGCCTGTCGGTGGTCATGCCGGTGGGTGGCCAGTCAAGCTTCTACTCCGACTGGTA
CCAGCCCGCCTGCGGCAAGGCCGGTTGCCAGACTTACAAGTGGGAGACCTTCCTGA
CCAGCGAGCTGCCGGGGTGGCTGCAGGCCAACAGGCACGTCAAGCCCACCGGAAGC
GCCGTCGTCGGTCTTTCGATGGCTGCTTCTTCGGCGCTGACGCTGGCGATCTATCACC
CCCAGCAGTTCGTCTACGCGGGAGCGATGTCGGGCCTGTTGGACCCCTCCCAGGCGA
TGGGTCCCACCCTGATCGGCCTGGCGATGGGTGACGCTGGCGGCTACAAGGCCTCC
GACATGTGGGGCCCGAAGGAGGACCCGGCGTGGCAGCGCAACGACCCGCTGTTGAA
CGTCGGGAAGCTGATCGCCAACAACACCCGCGTCTGGGTGTACTGCGGCAACGGCA
AGCCGTCGGATCTGGGTGGCAACAACCTGCCGGCCAAGTTCCTCGAGGGCTTCGTGC
GGACCAGCAACATCAAGTTCCAAGACGCCTACAACGCCGGTGGCGGCCACAACGGC

GTGTTCGACTTCCCGGACAGCGGTACGCACAGCTGGGAGTACTGGGGCGCGCAGCT
CAACGCTATGAAGCCCGACCTGCAACGGGCACTGGGTGCCACGCCCAACACCGGGC
CCGCGCCCCAGGGCGCCTAGTTTCTTAAGTTT

### Underlined sequences:

Start and stop codons
Afl II Restriction Site (NEB Buffer 4)
Note: multi T Spacer between stop and Afl II RE
Apa I Restriction Site (NEB Buffer 4)
Note: Spacer between start and Apa I RE is Kozak minimal translation initiation site

### Bold sequences:

HA 1 sequence bolded and underlined (SEQ ID NO 72)
HA2 sequence bolded (SEQ ID NO 73
NA1 sequence bolded and underlined (SEQ ID NO 74)

| | |
|---|---|
| SEQ ID NO 72 | GGGAATCTAttcATTGCTCCT |
| SEQ ID NO 73 | TGGGGGGTTattCACCACCCG |
| SEQ ID NO 74 | CATTATGAGGAATGTTCCTGTTAC |
| SEQ ID NO 75 | SEFAYGSFVRTVSLPVGADE (Heat Shock protein sequence of epitope HSPx derived from Mycobacterium tuberculosis H37Rv (NC_000962.2) |
| SEQ ID NO 76 | TCGGAATTCGCGTACGGTTCCTTCGTTCGCACGGTGTCGCTGC CGGTAGGTGCTGACGAG (nucleotide sequence corresponding to HSPx; SEQ ID NO 66) |
| SEQ ID NO 77 | GNLFIAPWGVIHHPHYEECSCY (underlined sequences are epitopes HA {composite} and NA, respectively, of Influenza A; Pep 11) |
| SEQ ID NO 78 | GGGAATCTAttcATTGCTCCTTGGGGGGTTattCACCACCCG CATTATGAGGAATGTTCCTGTTAC (Pep 11 - SEQ ID NO 68) |

SEQ ID NO 79 TCGGAATTCGCGTACGGTTCCTTCGTTCGCACGGTGTCGCTGCCGGTAGGTGCTGAC GAGGGGAATCTAttcATTGCTCCTTGGGGGGTTattCACCACCCGCATTATGAGGAATGT TCCTGTTACTCCCGGCCGGGCTTGCCGGTGGAGTACCTGCAGGTGCCGTCGCCGTCG ATGGGCCGTGACATCAAGGTCCAATTCCAAAGTGGTGGTGCCAACTCGCCCGCCCTG TACCTGCTCGACGGCCTGCGCGCGCAGGACGACTTCAGCGGCTGGGACATCAACAC CCCGGCGTTCGAGTGGTACGACCAGTCGGGCCTGTCGGTGGTCATGCCGGTGGGTGG CCAGTCAAGCTTCTACTCCGACTGGTACCAGCCCGCCTGCGGCAAGGCCGGTTGCCA GACTTACAAGTGGGAGACCTTCCTGACCAGCGAGCTGCCGGGGTGGCTGCAGGCCA ACAGGCACGTCAAGCCCACCGGAAGCGCCGTCGTCGGTCTTTCGATGGCTGCTTCTT CGGCGCTGACGCTGGCGATCTATCACCCCCAGCAGTTCGTCTACGCGGGAGCGATGT CGGGCCTGTTGGACCCCTCCCAGGCGATGGGTCCCACCCTGATCGGCCTGGCGATGG GTGACGCTGGCGGCTACAAGGCCTCCGACATGTGGGGCCCGAAGGAGGACCCGGCG TGGCAGCGCAACGACCCGCTGTTGAACGTCGGGAAGCTGATCGCCAACAACACCCG CGTCTGGGTGTACTGCGGCAACGGCAAGCCGTCGGATCTGGGTGGCAACAACCTGC CGGCCAAGTTCCTCGAGGGCTTCGTGCGGACCAGCAACATCAAGTTCCAAGACGCC TACAACGCCGGTGGCGGCCACAACGGCGTGTTCGACTTCCCGGACAGCGGTACGCA CAGCTGGGAGTACTGGGGCGCGCAGCTCAACGCTATGAAGCCCGACCTGCAACGGG CACTGGGTGCCACGCCCAACACCGGGCCCGCGCCCCAGGGCGCC (1008 nucleotide DNA construct of composite peptide comprising TB epitopes at each end with an influenza sequence in the middle which is composed of three epitopes - 2 HA composites {underlined} with an NA epitope between them).

| | |
|---|---|
| SEQ ID NO 80 | CAGAGNFIAP |
| SEQ ID NO 81 | CAGAGNLIAP |
| SEQ ID NO 82 | CAGAGNLFIAP |
| SEQ ID NO 83 | CAGAWGVHHP |
| SEQ ID NO 84 | CAGAWGIHHP |
| SEQ ID NO 85 | CAGAWGVIHHP |
| SEQ ID NO 86 | CAGAWGIVHHP |
| SEQ ID NO 87 | GNLIAPWGVIHHP |
| SEQ ID NO 88 | CAGAGNLIAPWGVIHHP |
| SEQ ID NO 89 | GNLFIAPWGVIHHP |
| SEQ ID NO 90 | CAGAGNLFIAPWGVIHHP |
| SEQ ID NO 91 | HYEECSCY |
| SEQ ID NO 92 | CAGAHYEECSCY |
| SEQ ID NO 93 | GNLFIAPWGVIHHPHYEECSCY |
| SEQ ID NO 94 | CAGAGNLFIAPWGVIHHPHYEECSCY |
| SEQ ID NO 95 | GNLFIAPWGVIHHPGNLFIAPWGVIHHP |
| SEQ ID NO 96 | CAGAGNLFIAPWGVIHHPGNLFIAPWGVIHHP |
| SEQ ID NO 97 | HYEECSCYGNLFIAPWGVIHHP |
| SEQ ID NO 98 | GNLFIAPHYEECSCYWGVIHHP |
| SEQ ID NO 99 | SLLTEVETPIRNEWGLLTEVETPIRQYIKANSKFIGITE |
| SEQ ID NO 100 | GNLFIAPGNLFIAPQYIKANSKFIGITEGNLFIAP |
| SEQ ID NO 101 | HYEECSCYDWSGYSGSFVQHPELTGLHYEECSCYQYIKANSKFIGITE |
| SEQ ID NO 102 | VTREPYVSCDPKSCINRCFYVELIRGRVTREPYVSCDPQYIKANSKFIGITE |
| SEQ ID NO 103 | DWSGYSGSFVQHPELTGL |
| SEQ ID NO 104 | ITGFAPFSKDNSIRLSAGGDIWVTREPYVSCDP |
| SEQ ID NO 105 | KSCINRCFYVELIRGR |
| SEQ ID NO 106 | GNLFIAPRYAFA |
| SEQ ID NO 107 | CAGAGNLFIAPRYAFA |
| SEQ ID NO 108 | GNLVVPRYAFA |
| SEQ ID NO 109 | CAGAGNLVVPRYAFA |
| SEQ ID NO 110 | GNLIAPRYAFA |
| SEQ ID NO 111 | CAGAGNLIAPRYAFA |
| SEQ ID NO 112 | GNLVVP |
| SEQ ID NO 113 | CAGAGNLVVP |
| SEQ ID NO 114 | FVIREPFISCSHLEC |
| SEQ ID NO 115 | CAGAFVIREPFISCSHLEC |

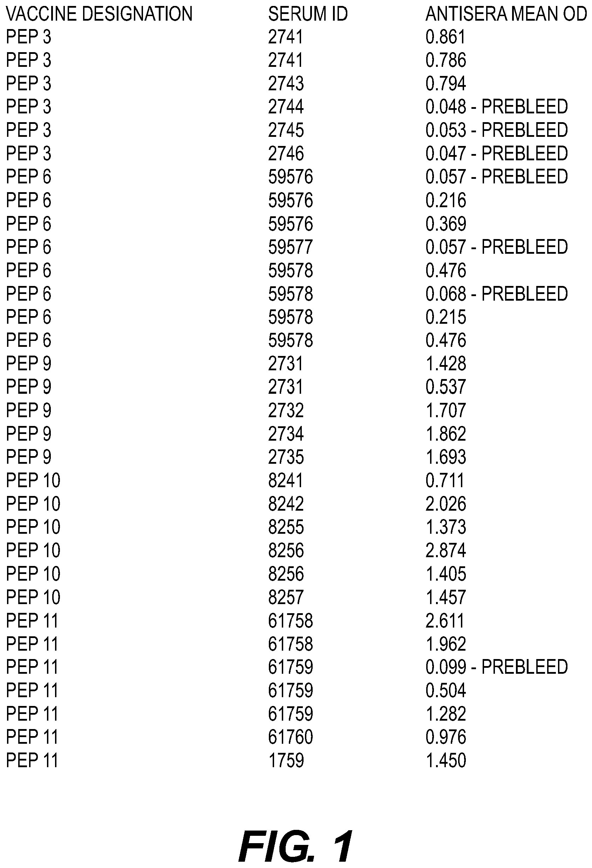

Figure 1 shows titers as determined by ELISA of mice vaccinated with Pep 6, Pep 9, Pep 10 or Pep 11. As can be seen, vaccinations with Pep 9, Pep 10 and Pep 11 provided a generally strong repose to the native antigen and the highest titers in mice.

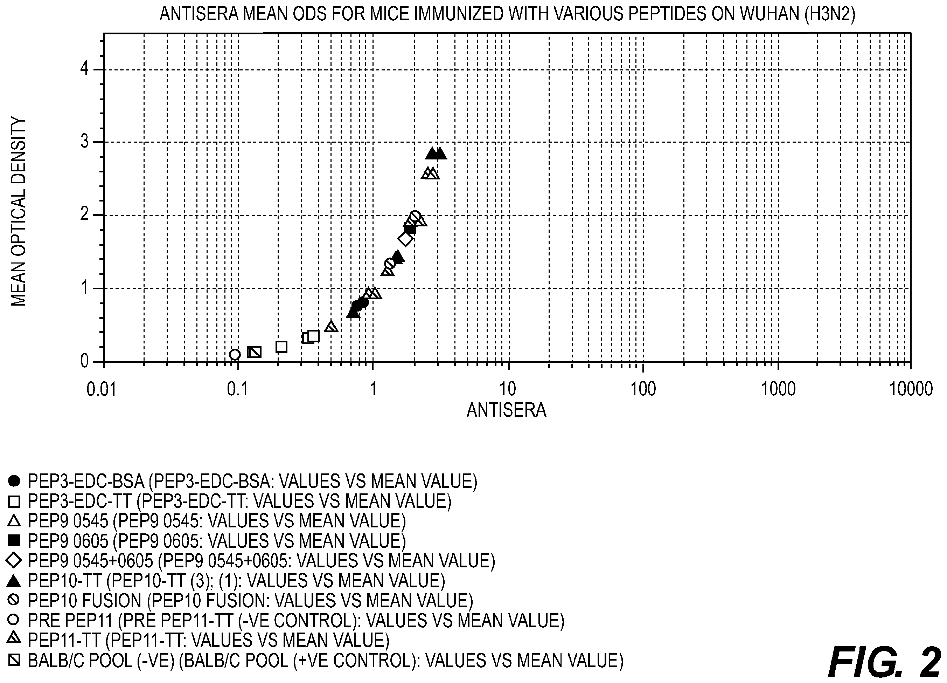

Figure 2 shows mean OD values of sera from mice immunized peptides derived from the Wuhan strain of Influenza (H3N2). Results indicate that Pep 10 and Pep 11 provide a significant immune response as compared to unvaccinated mice (the Balb/c pool) and mice vaccinated with Pep 3.

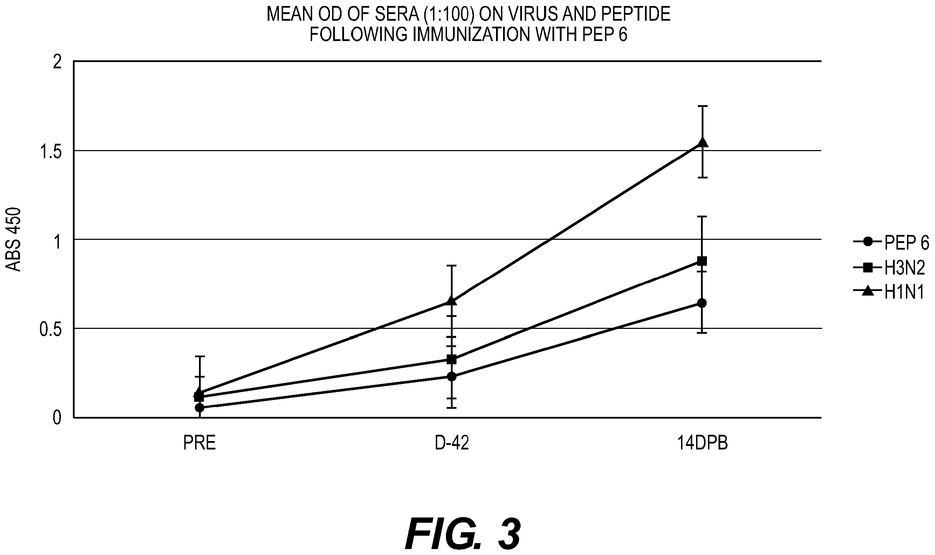

Figure 3 shows mean OD values of antisera (titered at 1:100) following immunization with Pep 6. As can be seen, Pep 6 the antisera reacted strongly with viruses of both H1N1 and H3N2.

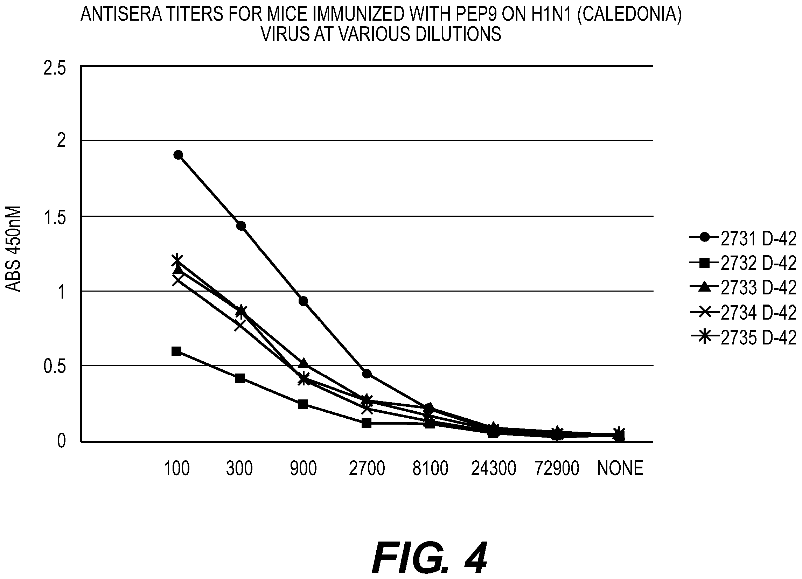

Figure 4 shows antisera titers of mice immunized with Pep 9 as reacted with Influenza strain Caledonia virus (H1N1) at various dilutions. As seen, Pep 9 reacts strongly with whole virus.

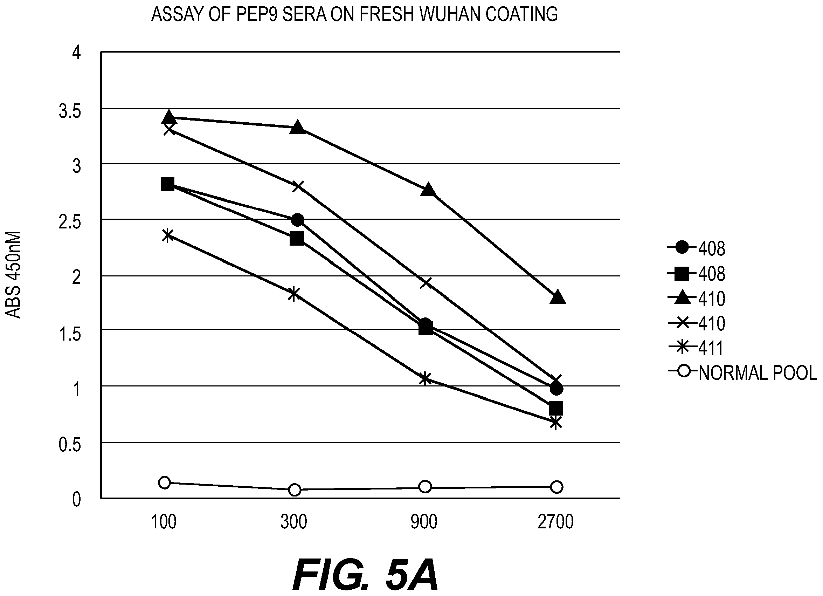

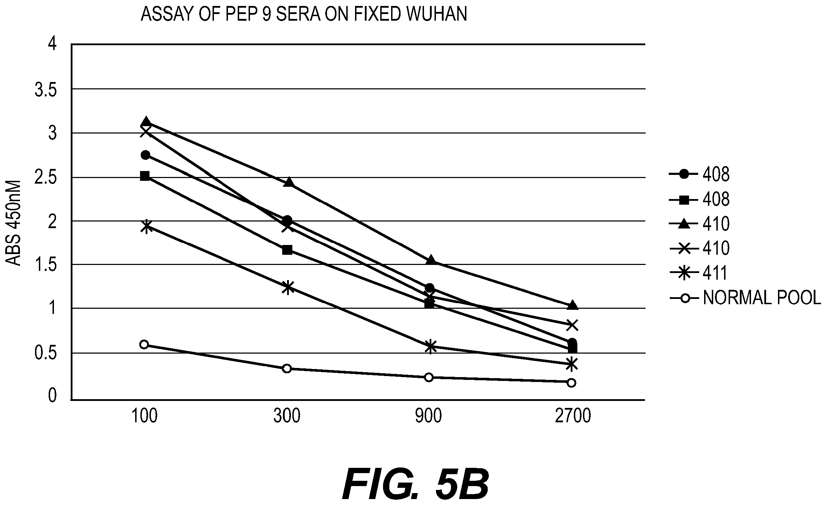

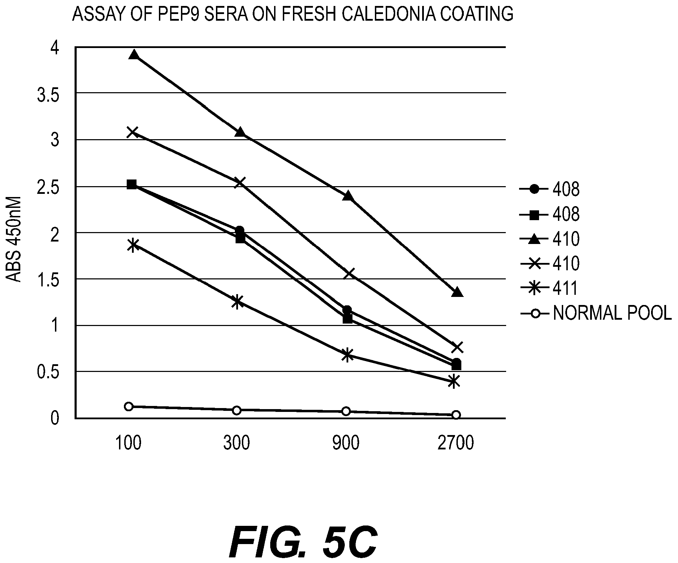

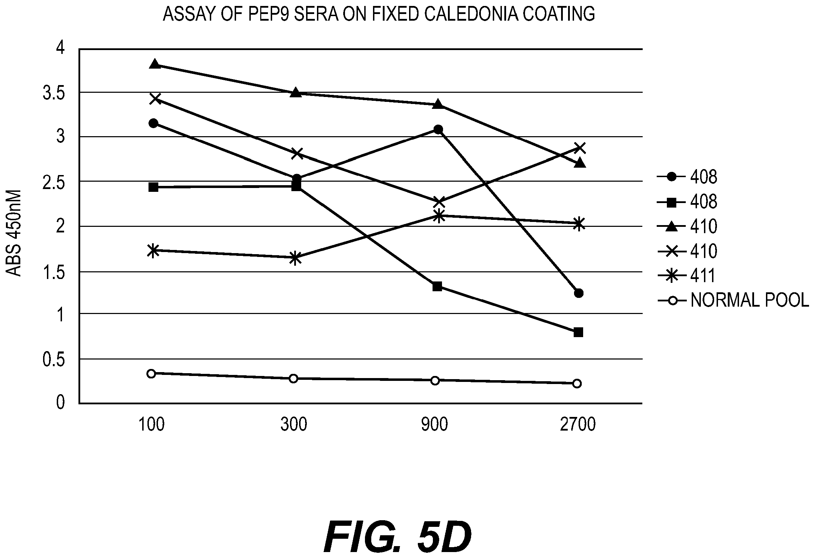

Figure 5 shows four graphs (A-D), each depicting the absorbance of Pep 9 sera to one of four substrates: (A) fresh Wuhan virus; (B) fixed Wuhan virus; (C) fresh Caledonia virus; and (D) fixed Caledonia virus. As shown, Pep 9 sera reacting strongly in with all substrates tested.

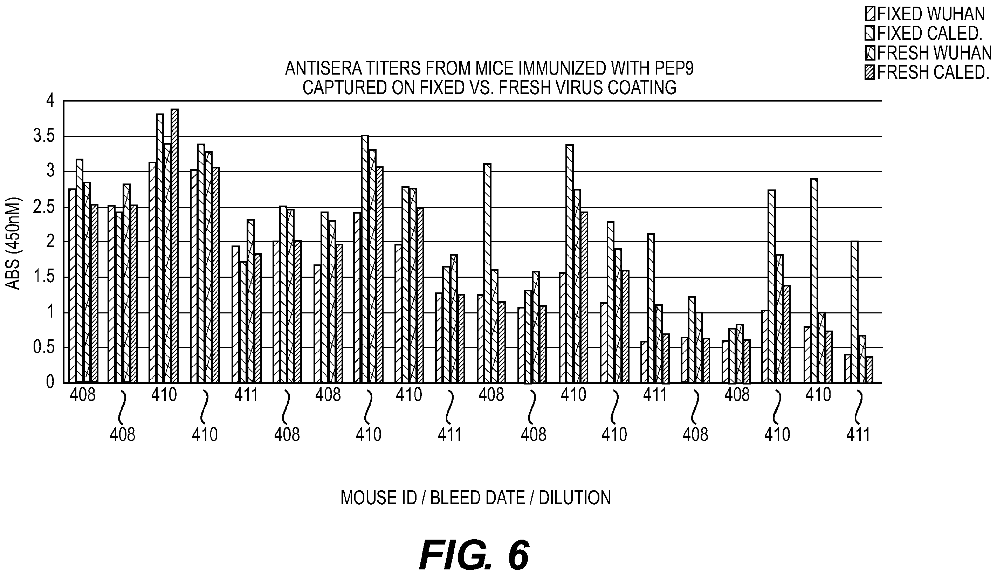

Figure 6 shows the titers from mice immunized with Pep 9 as captured with substrates of fresh or fixed Wuhan or Caledonia virus substrates. Again, Pep 9 antisera were strongly reactive with each and the amount of binding observed for many of the antisera tested was similar between fresh and fixed.

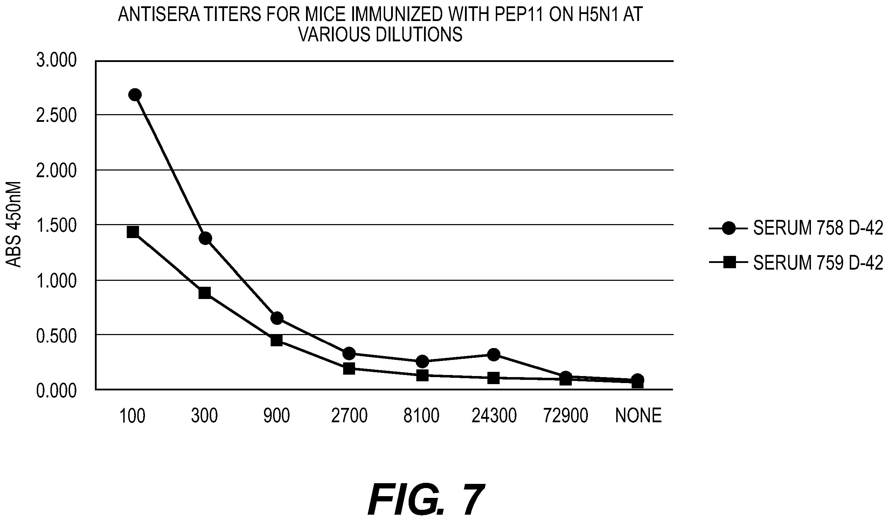

Figure 7 shows antisera titers of mice immunized with Pep 11 as captured with various dilutions of substrates of H5N1. As can be seen, binding was observed with both sera tested.

A composite antigen that contains previously determined conserved surface protein epitopes of hemagglutinin and neuraminidase from Influenza A viruses (several subtypes including human H1, H3 and high-path H5) was constructed. Also included were proteins segments from HspX and 85a from Mycobacterium tuberculosis. The expressed protein hybrid construct included the following: NH2+----HspX-HA1-HA2-NA1-85a-----COO-
wherein, HspX is 20 amino acids from Mycobacterium tuberculosis HspX protein; HA1 is a 7 amino acid highly conserved hybrid region influenza A hemagglutinin protein; HA2 is a 7 amino acid highly conserved hybrid region of influenza A hemagglutinin protein; NA1 is an 8 amino acid highly conserve hybrid region of influenza A neuraminidase protein; and 85a is 294 amino acids from the 85 kD 85a protein of Mycobacterium tuberculosis. The mature corresponding protein sequence is the 336 amino acid sequence of SEQ ID NO 70.

The composite antigen vaccine is constructed using the pVAX1 (Invitrogen Inc, Cat#V260-20) by recombinant methods. Briefly, a single stranded (ss) DNA polymer corresponding to SEQ ID 70 is synthesized. The ssDNA sequence also include: 1) a 5' ApaI restriction endonuclease recognition site and a 3' Afl II restriction site for directional insertion into pVAX1 cloning vector, 2) a ATT minimal Kozak translation initiation sequence at the 5' end, 3) a ATG start codon, and 4) a 3' TAG termination (stop) codon. The nucleotide sequence in its entirety will consist of 1038 nucleotides which is SEQ ID NO 71.

This single stranded nucleotide sequence is used as a template to generate double stranded amplicon by polymerase chain reaction. Following PCR amplification the amplicon is subjected to restriction endonuclease digestion, ligated into pVAX1 and transformed into *E. Coli* bacteria using standard transformation and screening methods with Kanamycin selection. Plasmids containing recombinant insert are grown in overnight culture and purified using known plasmid extraction methods. Concentrations of recombinant pVAX1 plasmid are subjected to DNA sequencing and utilized in downstream transfection experiments in mice.

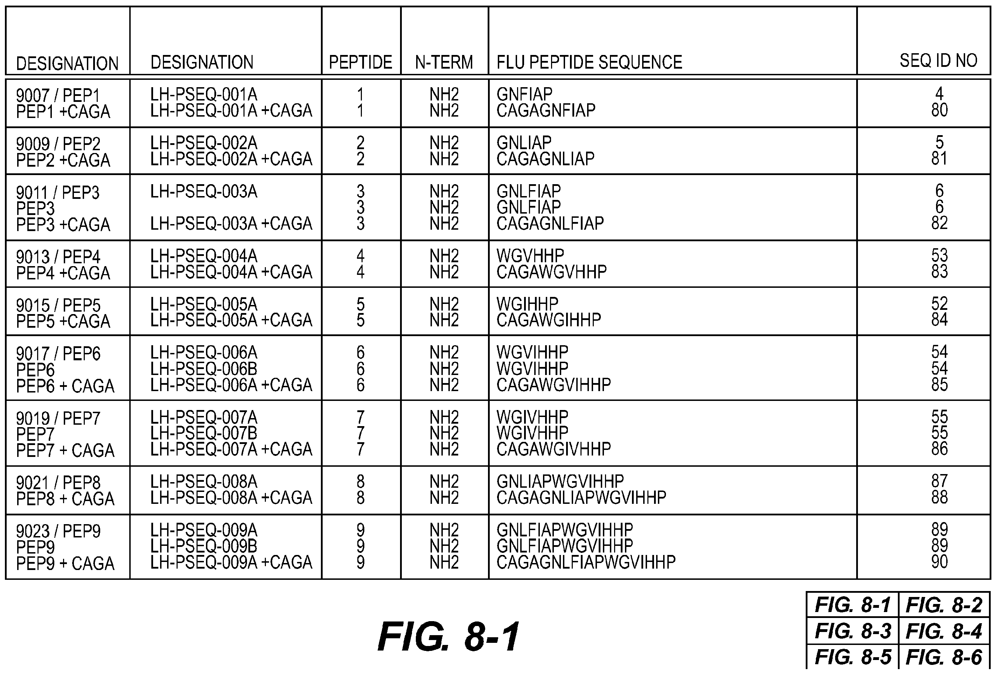

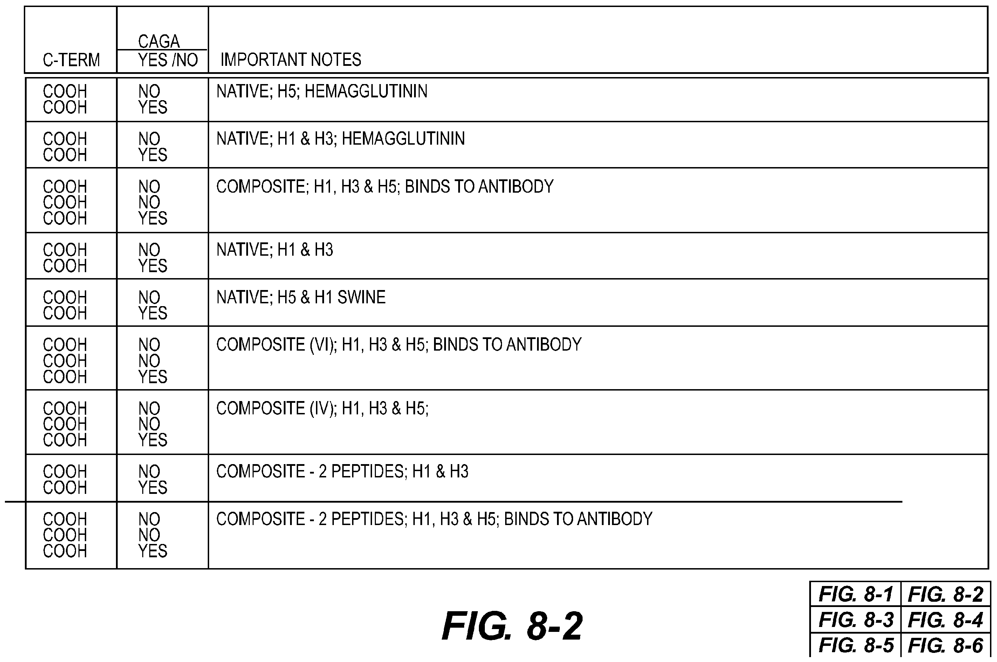

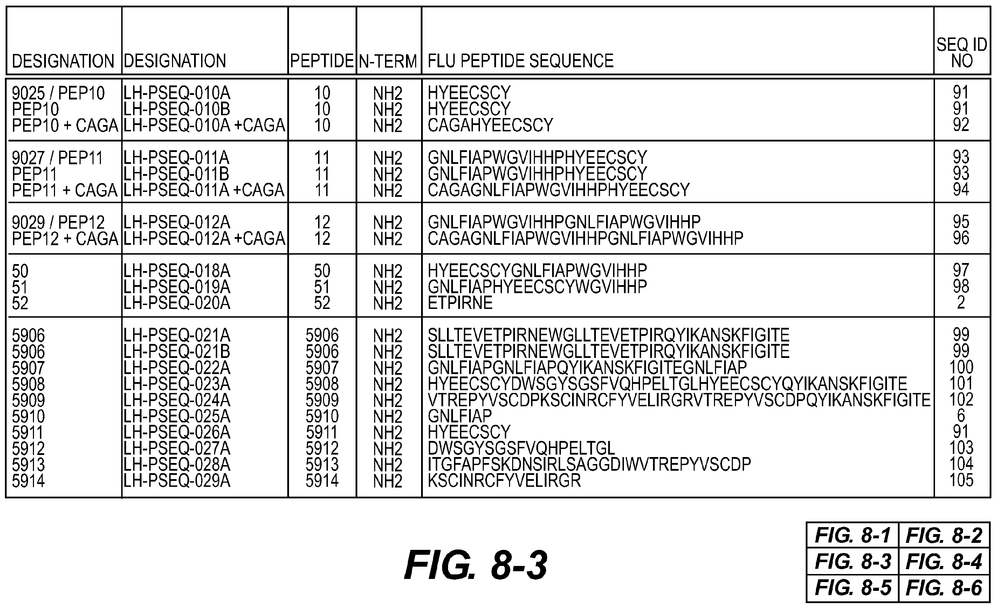

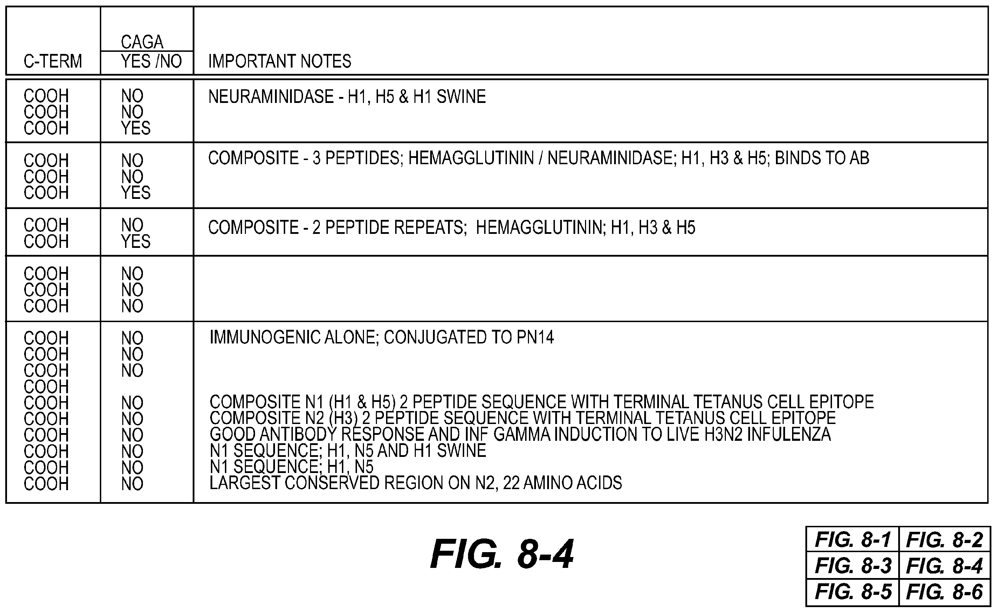

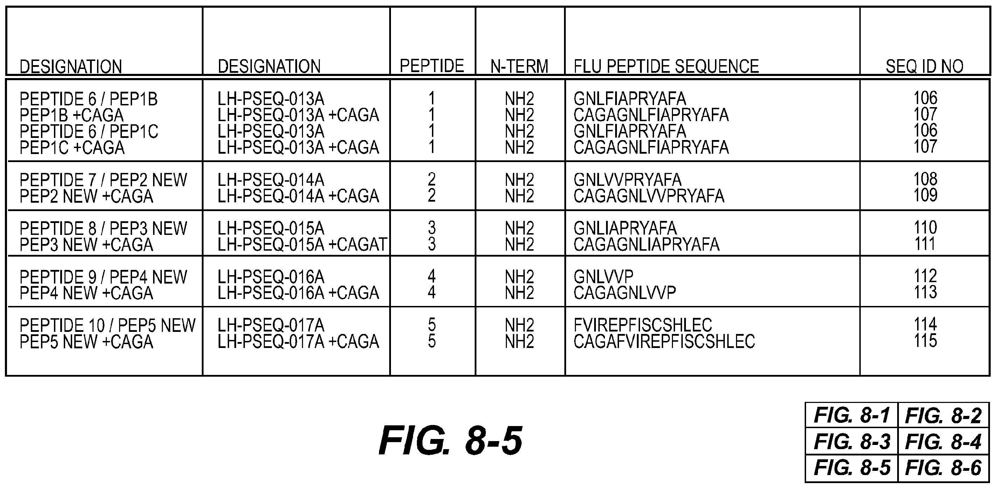

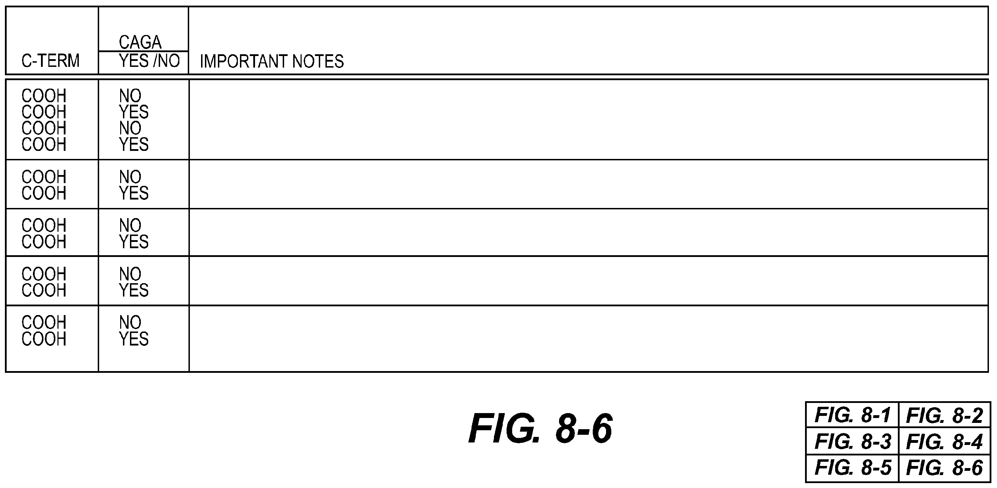

Figures 8-1 to 8-6 list a large number of sequences that include conserved or otherwise targeted regions of various genetic sequence of interest, and composite sequences of the disclosure.

## Claims

1. A composite antigen comprising SEQ ID NO: 66 or SEQ ID NO: 67.

2. The composite antigen of claim 1, wherein the composite antigen further comprises epitopes of proteins of Mycobacterium tuberculosis.

3. The composite antigen of claim 1, wherein the composite antigen comprises SEQ ID NO: 65 or 70.

4. The composite antigen of any one of the preceding claims, wherein the composite antigen further comprises epitopes of proteins of *Streptococcus, Pseudomonas, Shigella, Campylobacter, Salmonella, Haemophilus influenza, Chlamydophila pneumonia, Corynebacterium diphtheriae, Clostridium tetani, Mycoplasma pneumonia, Staphylococcus aureus, Moraxella catarrhalis, Legionella pneumophila, Bordetella pertussis* and *Escherichia coli.*

5. The composite antigen of claim 1, wherein the composite antigen comprises SEQ ID NO:94.

6. A recombinant polynucleotide that encodes the composite antigen of any one of claims 1 to 5.

7. The composite antigen of any one of claims 1 to 5 for use in a method for treating or preventing influenza in a mammal, the method comprising administering said composite antigen to the mammal.

8. A vaccine comprising the composite antigen of any one of claims 1 to 5.

9. A DNA vaccine that encodes the composite antigen of any one of claims 1 to 5.

## Patentansprüche

1. Zusammengesetztes Antigen, umfassend SEQ ID NO: 66 oder SEQ ID NO: 67.

2. Zusammengesetztes Antigen nach Anspruch 1, wobei das zusammengesetzte Antigen ferner Epitope von Proteinen von Mycobacterium tuberculosis umfasst.

3. Zusammengesetztes Antigen nach Anspruch 1, wobei das zusammengesetzte Antigen SEQ ID NO: 65 oder 70 umfasst.

4. Zusammengesetztes Antigen nach einem der vorhergehenden Ansprüche, wobei das zusammengesetzte Antigen ferner Epitope von Proteinen von *Streptococcus, Pseudomonas, Shigella, Campylobacter, Salmonella, Haemophilus influenza, Chlamydophila pneumonia, Corynebacterium diphtheriae, Clostridium tetani, Mycoplasma pneumonia, Staphylococcus aureus, Moraxella catarrhalis, Legionella pneumophila, Bordetella pertussis* und *Escherichia coli* umfasst.

5. Zusammengesetztes Antigen nach Anspruch 1, wobei das zusammengesetzte Antigen SEQ ID NO: 94 umfasst.

6. Rekombinantes Polynukleotid, das für das zusammengesetzte Antigen nach einem der Ansprüche 1 bis 5 kodiert.

7. Zusammengesetztes Antigen nach einem der Ansprüche 1 bis 5 zur Verwendung in einem Verfahren zur Behandlung oder Vorbeugung von Influenza bei einem Säugetier, wobei das Verfahren das Verabreichen des zusammengesetzten Antigens an das Säugetier umfasst.

8. Impfstoff, umfassend das zusammengesetzte Antigen nach einem der Ansprüche 1 bis 5.

9. DNA-Impfstoff, der für das zusammengesetzte Antigen nach einem der Ansprüche 1 bis 5 kodiert.

## Revendications

1. Antigène composite comprenant un identificateur de séquence SEQ ID N° 66 ou un identificateur de séquence SEQ ID N° 67.

2. Antigène composite selon la revendication 1, dans lequel l'antigène composite comprend en outre des épitopes de protéines de Mycobacterium tuberculosis.

3. Antigène composite selon la revendication 1, dans lequel l'antigène composite comprend un identificateur de séquence SEQ ID N° 65 ou un identificateur de séquence SEQ ID N° 70.

4. Antigène composite selon l'une quelconque des revendications précédentes, dans lequel l'antigène composite comprend en outre des épitopes de protéines de *Streptocoque, Pseudomonas, Shigella, Campylobacter, Salmonelle, Haemophilus influenza, Chlamydophila pneumonia, Corynebacterium diphtheriae, Clostridium tetani, Mycoplasma pneumonia, Staphylococcus aureus, Moraxella catarrhalis, Legionella pneumophila, Bordetella pertussis* et *Escherichia coli.*

5. *Antigène composite selon la revendication 5,* dans lequel l'antigène composite comprend un identificateur de séquence SEQ ID N° 94.

6. Polynucléotide recombinant qui code l'antigène composite selon l'une quelconque des revendications 1 à 5.

7. Antigène composite selon l'une quelconque des revendications 1 à 5 aux fins d'utilisation dans un procédé de traitement ou de prévention contre la grippe chez un mammifère, le procédé consistant à administrer ledit antigène composite au mammifère.

8. Vaccin contenant l'antigène composite selon l'une quelconque des revendications 1 à 5.

9. Vaccin ADN qui code l'antigène composite selon l'une quelconque des revendications 1 à 5.
